# EUROPEAN PATENT APPLICATION

(11) **EP 4 692 052 A1**
(43) Date of publication of application: **11.02.2026**
(21) Application number: 24810090.1
(22) Date of filing: 17.04.2024
(51) Int. Cl.: C07C 315/04, C07C 317/46, C07C 317/24, C07D 307/12, A01P 13/00

(54) **PREPARATION METHOD FOR 1-[2-CHLORO-3-(BROMOMETHYL)-4-(METHYLSULFONYL)PHENYL]ETHANONE AND THE USE OF SAME**

(30) Priority: 23.05.2023 CN 202310580010
(71) Applicant: Nutrichem Company Limited, Changping Dist., Beijing 102206 (CN)
(72) Inventor: ZHANG, Sheng, Beijing 102206 (CN); FAN, Shengyong, Beijing 102206 (CN); GUO, Yani, Beijing 102206 (CN); WU, Kun, Beijing 102206 (CN); WU, Guolin, Beijing 102206 (CN); WANG, Lei, Beijing 102206 (CN); WANG, Xiaolei, Beijing 102206 (CN); YANG, Haijian, Beijing 102206 (CN)
(74) Representative: reuteler & cie SA
(86) International application number: PCT/CN2024/088238
(87) International publication number: WO 2024/239843

(57) **Abstract**

Provided in the present invention is a preparation method for 1-[2-chloro-3-(bromomethyl)-4-(methylsulfonyl)phenyl]ethanone. A preparation method for 2-chloro-3-(trifluoroethoxymethyl)-4-(methylsulfonyl)benzoic acid of the present invention comprises: (1) in the presence of an initiator, subjecting 1-[2-chloro-3-(methyl)-4-(methylsulfonyl)phenyl]ethanone to a bromination reaction to obtain 1-[2-chloro-3-(bromomethyl)-4-(methylsulfonyl)phenyl]ethanone; (2) reacting 1-[2-chloro-3-(bromomethyl)-4-(methylsulfonyl)phenyl]ethanone to obtain 1-[2-chloro-3-(trifluoroethoxymethyl)-4-(methylsulfonyl)phenyl]ethanone; and (3) preparing 2-chloro-3-(trifluoroethoxymethyl)-4-(methylsulfonyl)benzoic acid from 1-[2-chloro-3-(trifluoroethoxymethyl)-4-(methylsulfonyl)phenyl]ethanone. The technical solution of the present invention has the advantages of involving few steps and achieving high yield, low cost and low energy consumption and the like, thus greatly reducing the production cost of tembotrione and tefuryltrione, and being more suitable for industrial production.

## Description

### Cross Reference to Related Applications

This application claims priority to Chinese patent application 202310580010.6, filed on May 23, 2023, which is specifically and entirely incorporated by reference.

### Field of the Invention

The present invention relates to the field of pesticide preparation, in particular to a preparation method for 1-[2-chloro-3-(bromomethyl)-4-(methylsulfonyl)phenyl]ethanone and use of the same.

### Background of the Invention

2-Chloro-3-(trifluoroethoxymethyl)-4-(methylsulfonyl)benzoic acid is an important intermediate in the preparation of a pesticidal herbicide tembotrione.

Tembotrione is a triketone corn field herbicide developed by Bayer Corporation in 2007, is a new compound synthesized by adding trifluoroethoxy on the basis of sulcotrione, belongs to a 4-hydroxyphenyl-pyruvate dioxygenase (HPPD) inhibitor, is currently one of the most important targets of herbicidal action, and has higher activity than sulcotrione and mesotrione, and compared with other varieties, tembotrione has a strong killing effect on a variety of weeds, no residual activity, strong resistance to rain erosion, and a broader herbicidal spectrum.

2-Chloro-3-[(*RS*-tetrahydrofuran-2-ylmethoxy)methyl)]-4-(methylsulfonyl)benzoic acid is an important intermediate in the synthesis of tefuryltrione.

Tefuryltrione is a benzoylcyclohexanedione herbicide co-developed by Bayer, Hokko Chemical and the National Federation of Agricultural Cooperative Associations. Tefuryltrione is a hot spot product for controlling weeds in rice fields in the future. Tefuryltrione belongs to a triketone herbicide and is a 4-hydroxyphenyl-pyruvate dioxygenase (HPPD) inhibitor, and HPPD is a key enzyme in biosynthetic pathways of plastoquinone and tocopherol essential for normal plant growth, and can catalyze the biochemical process from tyrosine to plastoquinone in plants. Tefuryltrione is absorbed mainly by leaves and roots and is conducted apically and basically in xylem and phloem, and is distributed throughout the plant. After weeds are treated with tefuryltrione, the leaf surface will become whitened, and then a meristem will become necrotic.

Tembotrione differs structurally from tefuryltrione by one group, and previous intermediates are the same except that trifluoroethanol is used for tembotrione and tetrahydrofurfuryl alcohol is used for tefuryltrione during etherification of a bromide with a metal salt of an alcohol.

At present, main synthetic routes of a known synthetic process involving a tembotrione intermediate 2-chloro-3-(trifluoroethoxymethyl)-4-(methylsulfonyl)benzoic acid is as follows:

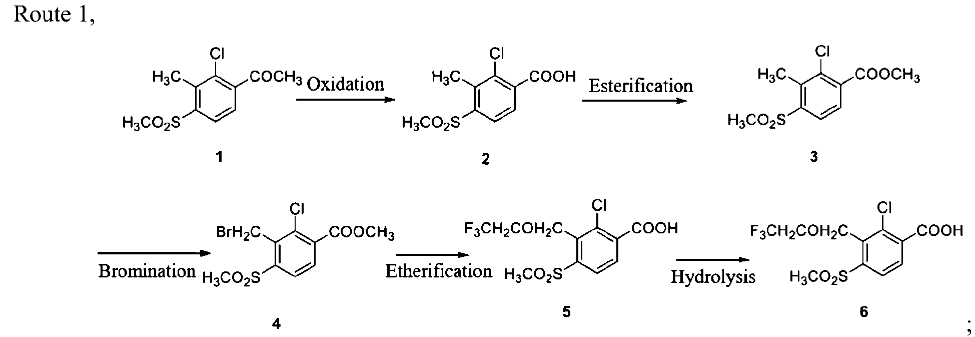

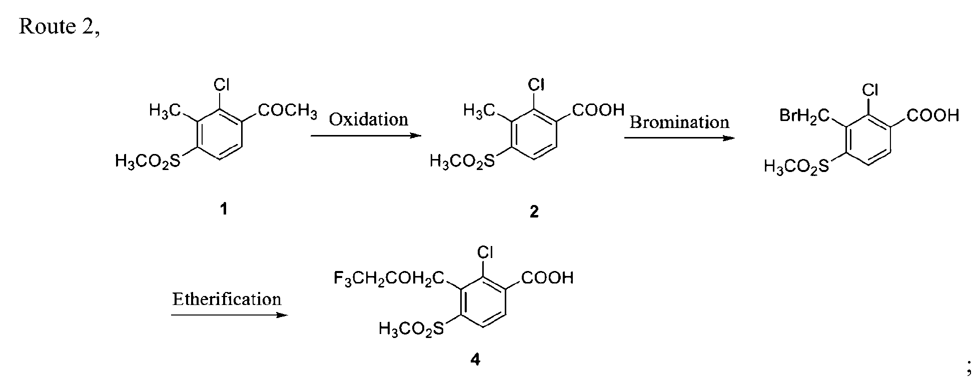

The above route 1 refers to a document 1 (Weijie Mi, Research on the Synthesis of tembotrione, Master's Thesis, Hebei University of Science and Technology, December 31, 2021). In the above reported route, 1-[2-chloro-3-(methyl)-4-(methylsulfonyl)phenyl]ethanone undergoes a haloform reaction or oxidation to obtain 2-chloro-3-(methyl)-4-(methylsulfonyl)benzoic acid, followed by esterification, bromination, trifluoromethoxy etherification, hydrolysis and the like to obtain a target intermediate 2-chloro-3-(trifluoroethoxymethyl)-4-(methylsulfonyl)benzoic acid. This process route has many synthetic steps, wherein the esterification is more difficult, and cannot be completed by a general esterification solution, and an ester is hydrolyzed into carboxylic acid after esterification in the above route, resulting in waste of resources and increasing the output of three wastes.

The route 2 refers to a document 2 (WO2003022800, Bayer, Method for producing 3-bromomethylbenzoic acids). Although this route omits two steps of esterification and hydrolysis, NBS is required during bromination due to the presence of carboxyl, a conventional bromination process cannot be used and the cost is high; and at least one more equivalent of the metal salt of the alcohol is required for the carboxyl to be consumed during etherification, resulting in waste and high cost.

The existing synthetic route of a tefuryltrione intermediate can refer to the above method using metal tetrahydrofurfuryl alcoholate during etherification.

In summary, the preparation of an intermediate of tembotrione or tefuryltrione 2-chloro-3-(trifluoroethoxymethyl)-4-(methylsulfonyl)benzoic acid or 2-chloro-3-[(RS-tetrahydrofuran-2-ylmethoxy)methyl]-4-(methylsulfonyl)benzoic acid in the prior art has the disadvantages of cumbersome steps, high energy consumption, high cost, low yield and the like.

### Summary of the Invention

An object of the present invention is to overcome the disadvantages of the synthetic routes in the prior art and provide a preparation method for 1-[2-chloro-3-(bromomethyl)-4-(methylsulfonyl)phenyl]ethanone and use of 1-[2-chloro-3-(bromomethyl)-4-(methylsulfonyl)phenyl]ethanone in the preparation of a tembotrione intermediate 2-chloro-3-(trifluoroethoxymethyl)-4-(methylsulfonyl)benzoic acid and a tefuryltrione intermediate 2-chloro-3-[(*RS*-tetrahydrofuran-2-ylmethoxy)methyl]-4-(methylsulfonyl)benzoic acid. In order to achieve the above object, the present invention provides a preparation method for 2-chloro-3-(trifluoroethoxymethyl)-4-(methylsulfonyl)benzoic acid, including:
Step (1), subjecting 1-[2-chloro-3-(methyl)-4-(methylsulfonyl)phenyl]ethanone to a bromination reaction with a brominating agent in the presence of an initiator to obtain 1-[2-chloro-3-(bromomethyl)-4-(methylsulfonyl)phenyl]ethanone;
Step (2), reacting 1-[2-chloro-3-(bromomethyl)-4-(methylsulfonyl)phenyl]ethanone with metal trifluoroethoxide to obtain 1-[2-chloro-3-(trifluoroethoxymethyl)-4-(methylsulfonyl)phenyl]ethanone; and
Step (3), preparing 2-chloro-3-(trifluoroethoxymethyl)-4-(methylsulfonyl)benzoic acid from 1-[2-chloro-3-(trifluoroethoxymethyl)-4-(methylsulfonyl)phenyl]ethanone.

Wherein in the step (3), 1-[2-chloro-3-(trifluoroethoxymethyl)-4-(methylsulfonyl)phenyl]ethanone may be subjected to an oxidation reaction with an oxidizing agent to obtain 2-chloro-3-(trifluoroethoxymethyl)-4-(methylsulfonyl)benzoic acid. 1-[2-Chloro-3-(trifluoroethoxymethyl)-4-(methylsulfonyl)phenyl]ethanone can also be subjected to a haloform reaction, followed by acidification to obtain 2-chloro-3-(trifluoroethoxymethyl)-4-(methylsulfonyl)benzoic acid.

The present invention also discloses a preparation method for 2-chloro-3-[(*RS*-tetrahydrofuran-2-ylmethoxy)methyl]-4-(methylsulfonyl)benzoic acid, including:
Step (1), subjecting 1-[2-chloro-3-(methyl)-4-(methylsulfonyl)phenyl]ethanone to a bromination reaction with a brominating agent in the presence of an initiator to obtain 1-[2-chloro-3-(bromomethyl)-4-(methylsulfonyl)phenyl]ethanone;
Step (2), reacting 1-[2-chloro-3-(bromomethyl)-4-(methylsulfonyl)phenyl]ethanone with metal tetrahydrofurfuryl alcoholate to obtain 1-{2-chloro-3-[(*RS*-tetrahydrofuran-2-ylmethoxy)methyl]-4-(methylsulfonyl)phenyl}ethanone; and
Step (3), preparing 2-chloro-3-[(*RS*-tetrahydrofuran-2-ylmethoxy)methyl]-4-(methylsulfonyl)benzoic acid from 1-{2-chloro-3-[(*RS*-tetrahydrofuran-2-ylmethoxy)methyl]-4-(methylsulfonyl)phenyl}ethanone.

The present invention also provides a compound of a formula (2),

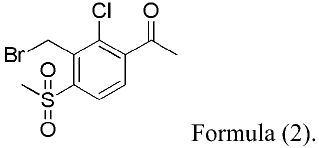

Preferably, the present invention provides a compound of a formula (3),

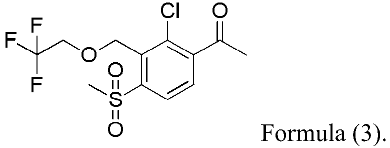

Preferably, the present invention also provides a compound of a formula (5),

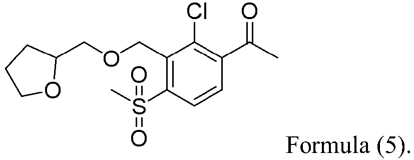

Further, the present invention discloses use of the compounds of the formula (2) and the formula (3) in the preparation of tembotrione.

Further, the present invention discloses use of the compounds of the formula (2) and the formula (5) in the preparation of tefuryltrione.

### Beneficial effects of the invention

1. In the present invention, the compound of the formula (3) or the compound of the formula (5) is prepared from the compound of the formula (2) for tembotrione and/or tefuryltrione, the esterification and hydrolysis steps in the prior art can be omitted, and at the same time, the defect of the harsh conditions of being required to change a reaction solvent and ensuring the anhydrous reaction of a system during the etherification reaction in the prior art is overcome. The technical solution of the present invention has the advantages of involving few steps and achieving high yield, low cost and low energy consumption and the like, thus greatly reducing the production cost of tembotrione and tefuryltrione, being more suitable for industrial production and having high competitiveness.

### Brief Description of Drawings

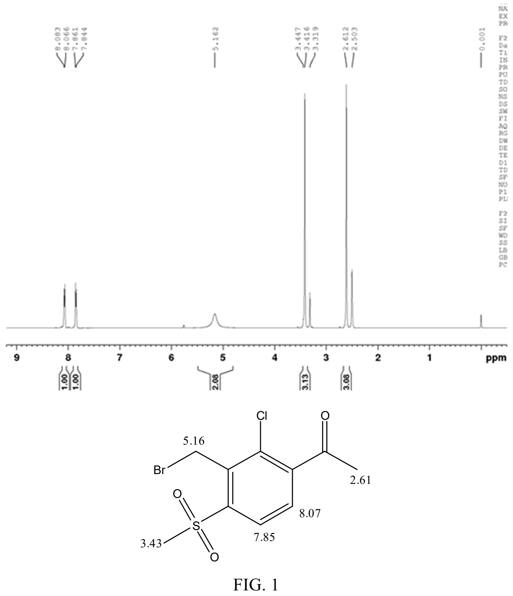

FIG. 1 is a nuclear magnetic resonance spectrum of 1-[2-chloro-3-(bromomethyl)-4-(methylsulfonyl)phenyl]ethanone (Formula (2)).

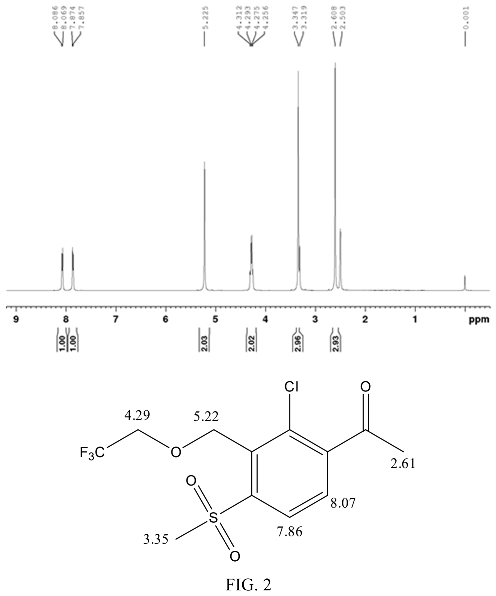

FIG. 2 is a nuclear magnetic resonance spectrum of 1-[2-chloro-3-(trifluoroethoxymethyl)-4-(methylsulfonyl)phenyl]ethanone (Formula (3)).

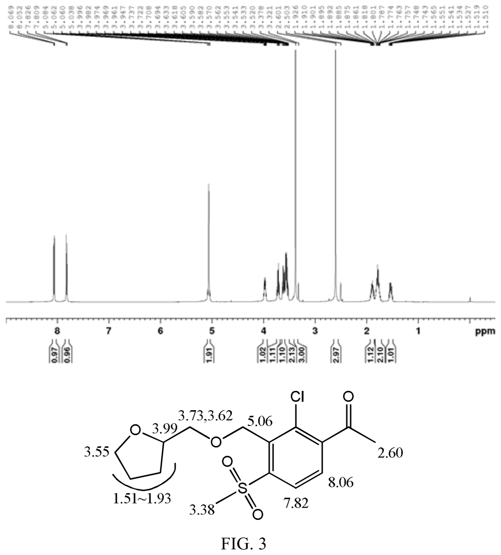

FIG. 3 is a nuclear magnetic resonance spectrum of 1-{2-chloro-3-[(*RS*-tetrahydrofuran-2-ylmethoxy)methyl]-4-(methylsulfonyl)phenyl}ethanone (Formula (5)).

### Detailed Description of the Embodiments

Specific embodiments of the present invention are further described below in detail with reference to the Examples. In order to make the objectives, technical solutions and advantages of the examples of the present invention more clear, the technical solutions in the examples of the present invention will be clearly and completely described below, and obviously, the described examples are a part of the examples of the present invention, rather than all of the examples. Based on the examples in the present invention, all other examples obtained by those of ordinary skill in the art without inventive step belong to the scope of protection of the present invention. Unless otherwise expressly indicated, throughout the description and claims, the term "include", or variations thereof such as "comprise" or "including" and the like will be understood to include the stated components or steps but not exclude the presence of other components or steps.

The endpoints and any values of the ranges disclosed herein are not limited to the precise range or value, and these ranges or values should be understood as including values close to these ranges or values. For numerical ranges, the endpoint values of each range, the endpoint values of each range and individual point values, and individual point values may be combined with each other to obtain one or more new numerical ranges, and these numerical ranges should be considered to be specifically disclosed herein.

In addition, numerous specific details are set forth in the following detailed description in order to better illustrate the present invention.

It should be understood by those skilled in the art that the present invention may also be implemented without certain specific details. In some examples, raw materials, methods, means and the like which are well known to those skilled in the art are not described in detail in order to highlight the subject matter of the present invention.

Hereinafter, the examples of the present invention are described in detail. However, these examples are exemplary, the present invention is not limited thereto, and the present invention is defined by the scope of the claims.

As used herein, the following terms used in the description and claims have the following meanings when specific definitions are not otherwise provided.

In the present invention, "solvent" refers to a liquid that dissolves a solid solute, a liquid solute, or a gaseous solute during a reaction. Examples of solvents of the present invention include one or more of water, 1,2-dichloroethane, chlorobenzene, acetic acid, dimethyl carbonate, ethylene glycol dimethyl ether and ethylene glycol diethyl ether, but are not limited to.

In the present invention, "initiator" refers to a free radical initiator, and refers to a class of compounds that are easily decomposed by heat into free radicals (i.e., primary free radicals). Examples of initiators in the present invention include azobisisobutyronitrile, azobisisoheptonitrile, benzoyl peroxide, and tert-butyl hydroperoxide, but are not limited thereto.

In the present invention, "brominating agent" refers to a reagent capable of providing bromine in a reaction, and examples of brominating agents include one or more of sodium bromate+sodium bromide+acid (a combination of sodium bromate, sodium bromide and an acid), hydrogen peroxide+sodium bromide+acid (a combination of hydrogen peroxide, sodium bromide and an acid), wherein the acid is sulfuric acid and/or phosphoric acid, hydrogen peroxide+hydrobromic acid (a combination of hydrogen peroxide and hydrobromic acid), sodium bromate+hydrobromic acid (a combination of sodium bromate and hydrobromic acid), liquid bromine, NBS (N-bromosuccinimide) and DBH (dibromohydantoin), but are not limited thereto.

Percentage in the present invention is generally understood according to the conventional meaning in the art, and generally refers to a percentage by weight.

In the present invention, 1-[2-chloro-3-(methyl)-4-(methylsulfonyl)phenyl]ethanone is as shown in a formula (1), 1-[2-chloro-3-(bromomethyl)-4-(methylsulfonyl)phenyl]ethanone is as shown in a formula (2), 1-[2-chloro-3-(trifluoroethoxymethyl)-4-(methylsulfonyl)phenyl]ethanone is as shown in a formula (3), 2-chloro-3-(trifluoroethoxymethyl)-4-(methylsulfonyl)benzoic acid is as shown in a formula (4), 1-{2-chloro-3-[(*RS*-tetrahydrofuran-2-ylmethoxy)methyl]-4-(methylsulfonyl)phenyl}ethanone is as shown in a formula (5), and 2-chloro-3-[(*RS*-tetrahydrofuran-2-ylmethoxy)methyl]-4-(methylsulfonyl)benzoic acid is as shown in a formula (6).

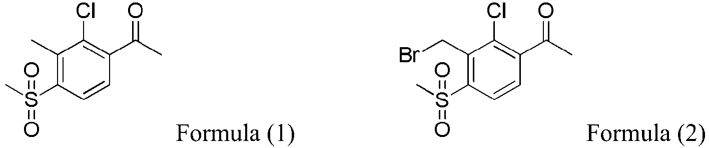

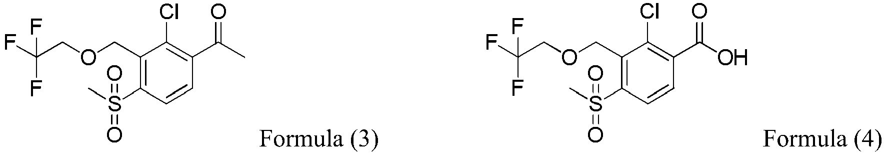

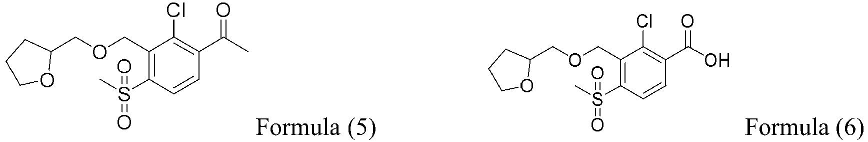

The present invention provides a preparation method for 2-chloro-3-(trifluoroethoxymethyl)-4-(methylsulfonyl)benzoic acid, including:
Step (1), subjecting 1-[2-chloro-3-(methyl)-4-(methylsulfonyl)phenyl]ethanone to a bromination reaction with a brominating agent in the presence of an initiator to obtain 1-[2-chloro-3-(bromomethyl)-4-(methylsulfonyl)phenyl]ethanone;
Step (2), reacting 1-[2-chloro-3-(bromomethyl)-4-(methylsulfonyl)phenyl]ethanone with metal trifluoroethoxide to obtain 1-[2-chloro-3-(trifluoroethoxymethyl)-4-(methylsulfonyl)phenyl]ethanone; and
Step (3), preparing 2-chloro-3-(trifluoroethoxymethyl)-4-(methylsulfonyl)benzoic acid from 1-[2-chloro-3-(trifluoroethoxymethyl)-4-(methylsulfonyl)phenyl]ethanone.

Its synthetic route is as follows:

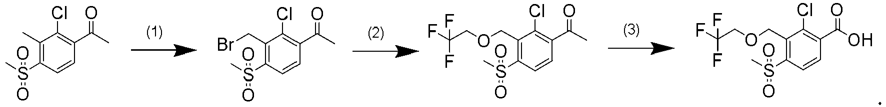

Preferably, in the above method, the reaction in the step (1) is carried out in a solvent selected from one or more of water, 1,2-dichloroethane, chlorobenzene, acetic acid, dimethyl carbonate, ethylene glycol dimethyl ether and ethylene glycol diethyl ether, preferably a combination of water and other solvents, particularly preferably a combination of chlorobenzene and water or a combination of 1,2-dichloroethane and water. It is found through the research that in the absence of the addition of water in a reaction solvent of the step (1), the effect of the reaction will become worse.

In the present invention, preferably, in the step (1), a solvent other than the water is used in an amount of 1000-2500 mL relative to 1 mol of 1-[2-chloro-3-(methyl)-4-(methylsulfonyl)phenyl]ethanone; and more preferably, the solvent other than the water is used in an amount of 1200-1500 mL relative to 1 mol of 1-[2-chloro-3-(methyl)-4-(methylsulfonyl)phenyl]ethanone.

In the present invention, preferably, in the step (1), the water is used in an amount of 100-1500 mL relative to 1 mol of 1-[2-chloro-3-(methyl)-4-(methylsulfonyl)phenyl]ethanone; more preferably, the water is used in an amount of 150-1000 mL relative to 1 mol of 1-[2-chloro-3-(methyl)-4-(methylsulfonyl)phenyl]ethanone; and further preferably, the water is used in an amount of 150-500 mL relative to 1 mol of 1-[2-chloro-3-(methyl)-4-(methylsulfonyl)phenyl]ethanone.

Particularly preferably, when the solvent in the step (1) is the combination of water and other solvents, a volume ratio of other solvents to the water is preferably 2-10:1, more preferably 3-8:1, further preferably 4-5:1.

Preferably, in the above method, the brominating agent in the step (1) is one or more of a combination of sodium bromate, sodium bromide and an acid (here, the acid is preferably sulfuric acid and/or phosphoric acid), a combination of hydrogen peroxide and sodium bromide and an acid (here, the acid is preferably sulfuric acid and/or phosphoric acid), a combination of hydrogen peroxide and hydrobromic acid, a combination of sodium bromate and hydrobromic acid, liquid bromine, NBS and DBH, preferably the combination of sodium bromate, sodium bromide and the acid (here, the acid is preferably sulfuric acid and/or phosphoric acid). A use method and useage amounts of sodium bromate+sodium bromide+acid (here, the acid is preferably sulfuric acid and/or phosphoric acid), hydrogen peroxide+sodium bromide+acid (here, the acid is preferably sulfuric acid and/or phosphoric acid), hydrogen peroxide+hydrobromic acid and sodium bromate+hydrobromic acid can adopt conventional amounts in the art when they are generally used as brominating agents. In addition, other brominating agents described above can also be used in amounts conventional in the art.

In the present invention, preferably, the brominating agent is used in an amount of 1-2 mol in terms of a bromine element relative to 1 mol of 1-[2-chloro-3-(methyl)-4-(methylsulfonyl)phenyl]ethanone; and more preferably, the brominating agent is used in an amount of 1-1.2 mol in terms of the bromine element relative to 1 mol of 1-[2-chloro-3-(methyl)-4-(methylsulfonyl)phenyl]ethanone.

In a specific embodiment of the present invention, the brominating agent is a combination of sodium bromate, sodium bromide and an acid, the acid is sulfuric acid and/or phosphoric acid, sodium bromate is used in an amount of 0.01-2 mol, sodium bromide is used in an amount of 0.01-2 mol, and the acid is used in an amount of 0.01-2 mol relative to 1 mol of 1-[2-chloro-3-(methyl)-4-(methylsulfonyl)phenyl]ethanone; and more preferably, sodium bromate is used in an amount of 0.4-0.8 mol, sodium bromide is used in an amount of 0.4-0.8 mol, and the acid is used in an amount of 0.4-0.8 mol relative to 1 mol of 1-[2-chloro-3-(methyl)-4-(methylsulfonyl)phenyl]ethanone.

The acid in the combination of sodium bromate, sodium bromide and the acid is preferably sulfuric acid, sulfuric acid is preferably used in the form of an aqueous solution, and the content of sulfuric acid in the aqueous sulfuric acid solution is preferably 20-50% by weight, preferably 30-40% by weight. That is, in the present invention, the combination of sodium bromate, sodium bromide and the acid is particularly preferably a combination of sodium bromate, sodium bromide and the aqueous sulfuric acid solution.

According to the present invention, preferably, the conditions of the bromination reaction include: a reaction temperature of 20-120°C and a reaction time of 3-10 h; and more preferably, the conditions of the bromination reaction include: a reaction temperature of 20-90°C and a reaction time of 3-6 h. According to the present invention, after the end of the bromination reaction, a target product can be obtained according to a conventional refining method in the art, and preferably, after the end of the bromination reaction, the resulting reaction solution is allowed to stand for liquid separation, an alkali is added into an organic phase for neutralization, and after the liquid separation, the organic phase is desolventized to obtain 1-[2-chloro-3-(bromomethyl)-4-(methylsulfonyl)phenyl]ethanone as a solid. The above alkali for neutralization is not particularly limited, and for example, may be sodium hydroxide and/or potassium hydroxide or the like.

According to the present invention, preferably, the initiator is one or more of azobisisobutyronitrile, azobisisoheptonitrile, benzoyl peroxide and tert-butyl hydroperoxide; and more preferably, the initiator is azobisisobutyronitrile. According to the present invention, preferably, the initiator is used in an amount of 0.01-0.5 mol relative to 1 mol of 1-[2-chloro-3-(methyl)-4-(methylsulfonyl)phenyl]ethanone; more preferably, the initiator is used in an amount of 0.02-0.1 mol relative to 1 mol of 1-[2-chloro-3-(methyl)-4-(methylsulfonyl)phenyl]ethanone; and further preferably, the initiator is used in an amount of 0.03-0.06 mol relative to 1 mol of 1-[2-chloro-3-(methyl)-4-(methylsulfonyl)phenyl]ethanone. It is found through the research that if the amount of the initiator used is too low, a good initiation effect can not be achieved.

Preferably, in the above method, the metal trifluoroethoxide in the step (2) is sodium trifluoroethoxide or potassium trifluoroethoxide; and the reaction in the step (2) is carried out in a solvent A which is one or more of tetrahydrofuran, 2-methyltetrahydrofuran, 1,2-dichloroethane, chlorobenzene, dimethyl carbonate, ethylene glycol dimethyl ether and ethylene glycol diethyl ether; more preferably one or more of chlorobenzene, ethylene glycol dimethyl ether and ethylene glycol diethyl ether; and particularly preferably chlorobenzene and/or ethylene glycol dimethyl ether. Preferably, the solvent A is used in an amount of 1000-3000 mL relative to 1 mol of 1-[2-chloro-3-(bromomethyl)-4-(methylsulfonyl)phenyl]ethanone; and more preferably, the solvent A is used in an amount of 1500-2500 mL relative to 1 mol of 1-[2-chloro-3-(bromomethyl)-4-(methylsulfonyl)phenyl]ethanone.

In a preferred embodiment of the present invention, the solvent A is the same as the solvent other than the water in the step (1).

Preferably, in the above method, sodium trifluoroethoxide and/or potassium trifluoroethoxide is used in an amount of 1-1.5 mol relative to 1 mol of 1-[2-chloro-3-(bromomethyl)-4-(methylsulfonyl)phenyl]ethanone; more preferably, sodium trifluoroethoxide and/or potassium trifluoroethoxide is used in an amount of 1-1.2 mol relative to 1 mol of 1-[2-chloro-3-(bromomethyl)-4-(methylsulfonyl)phenyl]ethanone; and further preferably, sodium trifluoroethoxide and/or potassium trifluoroethoxide is used in an amount of 1-1.1 mol relative to 1 mol of 1-[2-chloro-3-(bromomethyl)-4-(methylsulfonyl)phenyl]ethanone. Reaction studies have found that when the amount of sodium trifluoroethoxide and/or potassium trifluoroethoxide used is too high, an impurity in which a chlorine atom on a benzene ring is also substituted will appear in the reaction, and the impurity also increases as the amount of sodium trifluoroethoxide and/or potassium trifluoroethoxide used increases.

In the present invention, structural formulae of etherification of the bromobenzyl position, the disubstituted impurity in which a chlorine atom on the benzene ring is also substituted, and a hydrolysis impurity of bromobenzyl are as follows:

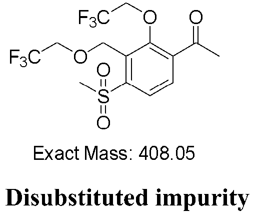

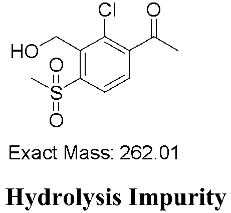

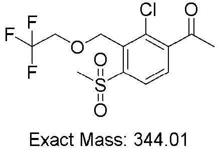

### Etherification of the bromobenzyl position (Formula 3).

In a preferred embodiment of the present invention, in the step (2), in a mixture of 1-[2-chloro-3-(bromomethyl)-4-(methylsulfonyl)phenyl]ethanone and a portion of a solvent, a mixture of sodium trifluoroethoxide and/or potassium trifluoroethoxide and another portion of the solvent is added dropwise. The another portion of the solvent is used such that the total content of sodium trifluoroethoxide and potassium trifluoroethoxide in the mixture of sodium trifluoroethoxide and/or potassium trifluoroethoxide and another portion of the solvent is 20-50% by weight, preferably 30-40% by weight.

In another preferred embodiment of the present invention, sodium trifluoroethoxide and/or potassium trifluoroethoxide is used in the form of an aqueous solution, and the content of sodium trifluoroethoxide and potassium trifluoroethoxide in the aqueous solution of sodium trifluoroethoxide and/or potassium trifluoroethoxide may be 20-50% by weight, preferably 30-40% by weight.

According to the present invention, preferably, in the step (2), the conditions of the reaction include: a reaction temperature of -10°C to 100°C and a reaction time of 3-10 h; and more preferably, the conditions of the reaction include: a reaction temperature of 0-25°C and a reaction time of 3-8 h.

According to the present invention, after the reaction in the step (2) is finished, a target product can be obtained according to a conventional refining method in the art, and preferably, after the reaction in the step (2) is finished, only an insoluble matter needs to be filtered, and a filtrate is desolventized under a negative pressure to obtain 1-[2-chloro-3-(trifluoroethoxymethyl)-4-(methylsulfonyl)phenyl]ethanone. In addition, in the case where water is used in the step (2) (that is, in the case where sodium trifluoroethoxide and/or potassium trifluoroethoxide is used in the form of an aqueous solution), after completion of the reaction, the resulting reaction solution is allowed to stand for layering, an organic phase is separated, and the organic phase is desolventized under a negative pressure to obtain 1-[2-chloro-3-(trifluoroethoxymethyl)-4-(methylsulfonyl)phenyl]ethanone.

The filtrate and the organic phase can also be used directly in a reaction of a next step, and hence, the step (2) and the reaction of the next step, i.e., the step (3) can be carried out in the same solvent, and thus, the step (1), the step (2) and the step (3) can be carried out in the same solvent, which is very suitable for industrial preparation.

Preferably, in the above method, 1-[2-chloro-3-(trifluoroethoxymethyl)-4-(methylsulfonyl)phenyl]ethanone is subjected to an oxidation reaction with an oxidizing agent to obtain 2-chloro-3-(trifluoroethoxymethyl)-4-(methylsulfonyl)benzoic acid in the step (3).

According to the present invention, in a preferred embodiment, the oxidation reaction in the step (3) is carried out in the presence of a catalyst, and the catalyst can adopt various catalysts generally used in the art for catalyzing the oxidation reaction, and for example, one or more of cobalt acetate, manganese acetate, copper acetate, vanadium pentoxide, selenium dioxide, ruthenium on carbon and ruthenium dioxide, preferably one or more of cobalt acetate, manganese acetate, selenium dioxide and ruthenium dioxide.

In the present invention, preferably, the catalyst is used in an amount of 0.01-0.5 mol relative to 1 mol of 1-[2-chloro-3-(trifluoroethoxymethyl)-4-(methylsulfonyl)phenyl]ethanone; more preferably, the catalyst is used in an amount of 0.02-0.3 mol relative to 1 mol of 1-[2-chloro-3-(trifluoroethoxymethyl)-4-(methylsulfonyl)phenyl]ethanone; and preferably, the catalyst is used in an amount of 0.05-0.1 mol relative to 1 mol of 1-[2-chloro-3-(trifluoroethoxymethyl)-4-(methylsulfonyl)phenyl]ethanone.

According to the present invention, preferably, the reaction in the step (3) is carried out in a solvent B which is one or more of chlorobenzene, toluene, acetic acid, tert-butanol, isopropanol, dimethyl carbonate, ethylene glycol dimethyl ether, ethylene glycol diethyl ether, diethylene glycol dimethyl ether and pyridine; and more preferably, the solvent B is one or more of chlorobenzene, toluene, ethylene glycol dimethyl ether, ethylene glycol diethyl ether and diethylene glycol dimethyl ether. In the present invention, preferably, the solvent B is used in an amount of 1000-3000 mL relative to 1 mol of 1-[2-chloro-3-(trifluoroethoxymethyl)-4-(methylsulfonyl)phenyl]ethanone; and more preferably, the solvent B is used in an amount of 1500-1800 mL relative to 1 mol of 1-[2-chloro-3-(trifluoroethoxymethyl)-4-(methylsulfonyl)phenyl]ethanone.

In a preferred embodiment of the present invention, the solvent B is the same as the solvent A.

In another preferred embodiment of the present invention, the solvent B is the same as the solvent other than the water in the step (1).

In another preferred embodiment of the present invention, the solvent B is the same as the solvent A and the solvent other than the water in the step (1).

According to the present invention, preferably, the oxidizing agent may be various oxidizing agents commonly used in the art, and may be, for example, one or more of oxygen, compressed air, ozone, hydrogen peroxide, nitric acid, manganese dioxide, sodium periodate and potassium periodate.

In the present invention, preferably, the oxidizing agent is used in an amount conventional in the art according to the specifically selected oxidizing agent. For example, the oxidizing agent is used in an amount of 1-10 mol relative to 1 mol of 1-[2-chloro-3-(trifluoroethoxymethyl)-4-(methylsulfonyl)phenyl]ethanone; and more preferably, the oxidizing agent is used in an amount of 1-5 mol relative to 1 mol of 1-[2-chloro-3-(trifluoroethoxymethyl)-4-(methylsulfonyl)phenyl]ethanone.

According to the present invention, preferably, the conditions of the oxidation reaction include: a reaction temperature of 20-250°C and a reaction time of 0.5-10 h; and more preferably, the conditions of the oxidation reaction include: a reaction temperature of 20-120°C and a reaction time of 2-5 h. According to the present invention, after the oxidation reaction is completed, a target product can be obtained according to a conventional refining method in the art, and preferably, a part of the solvent is removed from the reaction product, and the temperature is reduced for crystallization to obtain the target product 2-chloro-3-(trifluoroethoxymethyl)-4-(methylsulfonyl)benzoic acid.

Preferably, in the above method of the present invention, 1-[2-chloro-3-(trifluoroethoxymethyl)-4-(methylsulfonyl)phenyl]ethanone is subjected to a haloform reaction with a haloform reaction reagent, followed by acidification by addition of an aqueous solution of an acid to obtain 2-chloro-3-(trifluoroethoxymethyl)-4-(methylsulfonyl)benzoic acid in the step (3). According to the present invention, preferably, the haloform reaction reagent is sodium hypochlorite, sodium hypobromite or liquid bromine+sodium hydroxide; and more preferably, the haloform reaction reagent is sodium hypochlorite.

According to the present invention, preferably, the haloform reaction in the above method is carried out in a solvent C which is one or more of 1,2-dichloroethane, chloroform, carbon tetrachloride and chlorobenzene; and more preferably, the solvent C is 1,2-dichloroethane. Preferably, the solvent C is used in an amount of 1000-3000 mL relative to 1 mol of 1-[2-chloro-3-(trifluoroethoxymethyl)-4-(methylsulfonyl)phenyl]ethanone; and more preferably, the solvent C is used in an amount of 1500-1800 mL relative to 1 mol of 1-[2-chloro-3-(trifluoroethoxymethyl)-4-(methylsulfonyl)phenyl]ethanone.

In the present invention, preferably, the haloform reaction reagent is used in an amount of 3-4.5 mol in terms of effective halogen relative to 1 mol of 1-[2-chloro-3-(trifluoroethoxymethyl)-4-(methylsulfonyl)phenyl]ethanone; and more preferably, the haloform reaction reagent is used in an amount of 3-3.6 mol in terms of effective halogen relative to 1 mol of 1-[2-chloro-3-(trifluoroethoxymethyl)-4-(methylsulfonyl)phenyl]ethanone.

According to the present invention, preferably, the conditions of the haloform reaction include: a reaction temperature of 20-80°C and a reaction time of 0.5-10 h; and more preferably, the conditions of the haloform reaction include: a reaction temperature of 20-50°C and a reaction time of 3-5 h. According to the present invention, in the above preferred preparation method, the acid in the aqueous solution of the acid added after the haloform reaction may be an inorganic acid and an organic acid, and preferably, the acid is one or more of hydrochloric acid, sulfuric acid, acetic acid, formic acid and phosphoric acid; and more preferably, the acid is hydrochloric acid and/or sulfuric acid. In addition, the acidification is preferably such that a pH value of a reaction system is 3.5 or less. Specifically, the pH value can be, for example, 3.5, 3.4, 3.3, 3.2, 3.1, 3, 2.9, 2.8, 2.6, 2.4, 2.2, 2, 1.8, 1.6, 1.4, 1.2, 1, 0.5 or the like, and is in the range formed by any two of the above values.

According to the present invention, preferably, the acidification in the above preparation method is performed at a temperature of -30°C to 105°C; more preferably, the acidification is is performed at a temperature of 40-80°C; and further preferably, the acidification is performed at a temperature of 40-60°C. In a preferred embodiment of the present invention, the aqueous solution of the acid (e.g., sulfuric acid, hydrochloric acid, etc.) is added after the haloform reaction, so that the resulting reaction solution is neutralized to pH=6-7, the reaction solution is allowed to stand for liquid separation, an aqueous phase is separated and acidified at 40-50°C by addition of the aqueous solution of the acid to pH=3-3.3, stirring is then performed at 40-50°C for 1-5 h, acidification is performed at 40-50°C by addition of an aqueous solution of an acid to pH=1-2, and stirring is then performed at 40-50°C for 1-3 h.

According to the present invention, after the acidification is completed, a target product can be obtained according to a conventional refining method in the art, and preferably, the acidified product is subjected to solid-liquid separation to obtain the target product 2-chloro-3-(trifluoroethoxymethyl)-4-(methylsulfonyl)benzoic acid.

Preferably, the present invention provides a compound of a formula (2),

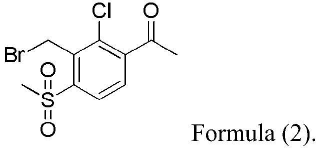

Preferably, the present invention also provides a compound of a formula (3),

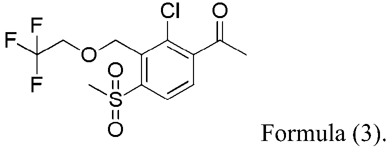

Particularly preferably, the present invention provides use of a compound of a formula (2) and a compound of a formula (3) in the preparation of 2-chloro-3-(trifluoroethoxymethyl)-4-(methylsulfonyl)benzoic acid, wherein structures of the formula (2) and the formula (3) are as follows:

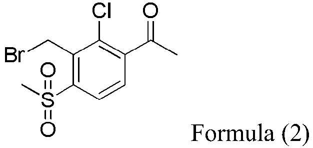

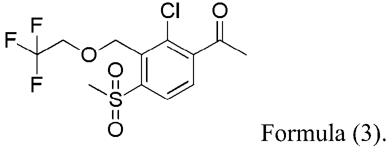

In the present invention, by the use of the compound of the formula (2) and the compound of the formula (3) in the preparation of 2-chloro-3-(trifluoroethoxymethyl)-4-(methylsulfonyl)benzoic acid, the process steps can be greatly shortened, the requirements of the reaction conditions are reduced, the process is more environmentally friendly, the production cost is reduced, and the reaction conditions are more suitable for industrialization.

The present invention also provides a preparation method for 2-chloro-3-[(RS-tetrahydrofuran-2-ylmethoxy)methyl]-4-(methylsulfonyl)benzoic acid, including:
Step (1), subjecting 1-[2-chloro-3-(methyl)-4-(methylsulfonyl)phenyl]ethanone to a bromination reaction with a brominating agent in the presence of an initiator to obtain 1-[2-chloro-3-(bromomethyl)-4-(methylsulfonyl)phenyl]ethanone;
Step (2), reacting 1-[2-chloro-3-(bromomethyl)-4-(methylsulfonyl)phenyl]ethanone with metal tetrahydrofurfuryl alcoholate to obtain 1-{2-chloro-3-[(*RS*-tetrahydrofuran-2-ylmethoxy)methyl]-4-(methylsulfonyl)phenyl}ethanone; and
Step (3), preparing 2-chloro-3-[(*RS*-tetrahydrofuran-2-ylmethoxy)methyl]-4-(methylsulfonyl)benzoic acid from 1-{2-chloro-3-[(*RS*-tetrahydrofuran-2-ylmethoxy)methyl]-4-(methylsulfonyl)phenyl}ethanone.

Its synthetic route is as follows:

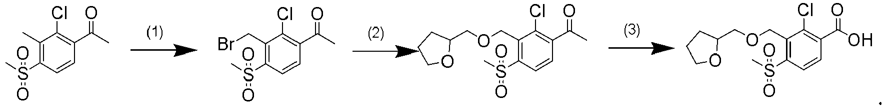

Preferably, in the above method, the reaction in the step (1) is carried out in a solvent selected from one or more of water, 1,2-dichloroethane, chlorobenzene, acetic acid, dimethyl carbonate, ethylene glycol dimethyl ether and ethylene glycol diethyl ether, preferably a combination of water and other solvents, particularly preferably a combination of chlorobenzene and water or 1,2-dichloroethane and water. It is found through the research that in the absence of the addition of water in a reaction solvent of the step (1), the effect of the reaction will become worse.

In the present invention, preferably, in the step (1), a solvent other than the water is used in an amount of 1000-2500 mL relative to 1 mol of 1-[2-chloro-3-(methyl)-4-(methylsulfonyl)phenyl]ethanone; and more preferably, the solvent other than the water is used in an amount of 1200-1500 mL relative to 1 mol of 1-[2-chloro-3-(methyl)-4-(methylsulfonyl)phenyl]ethanone.

In the present invention, preferably, in the step (1), the water is used in an amount of 100-1500 mL relative to 1 mol of 1-[2-chloro-3-(methyl)-4-(methylsulfonyl)phenyl]ethanone; more preferably, the water is used in an amount of 150-1000 mL relative to 1 mol of 1-[2-chloro-3-(methyl)-4-(methylsulfonyl)phenyl]ethanone; and further preferably, the water is used in an amount of 150-500 mL for 1 mol of 1-[2-chloro-3-(methyl)-4-(methylsulfonyl)phenyl]ethanone. Particularly preferably, when the solvent in the step (1) is the combination of water and other solvents, a volume ratio of other solvents to the water is preferably 2-10:1, more preferably 3-8:1, further preferably 4-5:1.

Preferably, in the above method, the brominating reagent in the step (1) is one or more of a combination of sodium bromate, sodium bromide and an acid, a combination of hydrogen peroxide and sodium bromide and an acid, a combination of hydrogen peroxide and hydrobromic acid, a combination of sodium bromate and hydrobromic acid, liquid bromine, NBS and DBH, preferably the combination of sodium bromate, sodium bromide and the acid, wherein the acid in the combination of sodium bromate, sodium bromide and the acid and the combination of hydrogen peroxide and sodium bromide and the acid is preferably sulfuric acid and/or phosphoric acid. A use method and useage amounts of sodium bromate+sodium bromide+acid, hydrogen peroxide+sodium bromide+acid, hydrogen peroxide+hydrobromic acid and sodium bromate+hydrobromic acid can adopt conventional amounts in the art when they are generally used as brominating agents. In addition, other brominating agents described above can also be used in amounts conventional in the art.

In the present invention, preferably, the brominating agent is used in an amount of 1-2 mol in terms of a bromine element relative to 1 mol of 1-[2-chloro-3-(methyl)-4-(methylsulfonyl)phenyl]ethanone; and more preferably, the brominating agent is used in an amount of 1-1.2 mol in terms of the bromine element relative to 1 mol of 1-[2-chloro-3-(methyl)-4-(methylsulfonyl)phenyl]ethanone.

In a specific embodiment of the present invention, the brominating agent is a combination of sodium bromate, sodium bromide and an acid, the acid is sulfuric acid and/or phosphoric acid, sodium bromate is used in an amount of 0.01-2 mol, sodium bromide is used in an amount of 0.01-2 mol, and the acid is used in an amount of 0.01-2 mol relative to 1 mol of 1-[2-chloro-3-(methyl)-4-(methylsulfonyl)phenyl]ethanone; and more preferably, sodium bromate is used in an amount of 0.4-0.8 mol, sodium bromide is used in an amount of 0.4-0.8 mol, and the acid is used in an amount of 0.4-0.8 mol relative to 1 mol of 1-[2-chloro-3-(methyl)-4-(methylsulfonyl)phenyl]ethanone.

The acid in the combination of sodium bromate, sodium bromide and the acid is preferably sulfuric acid, sulfuric acid is preferably used in the form of an aqueous solution, and the content of sulfuric acid in the aqueous sulfuric acid solution is preferably 20-50% by weight, preferably 30-40% by weight. That is, in the present invention, the combination of sodium bromate, sodium bromide and the acid is particularly preferably a combination of sodium bromate, sodium bromide and the aqueous sulfuric acid solution.

According to the present invention, preferably, the conditions of the bromination reaction include: a reaction temperature of 20-120°C and a reaction time of 3-10 h; and more preferably, the conditions of the bromination reaction include: a reaction temperature of 20-90°C and a reaction time of 3-6 h. According to the present invention, after the end of the bromination reaction, a target product can be obtained according to a conventional refining method in the art, and preferably, after the end of the bromination reaction, the resulting reaction solution is allowed to stand for liquid separation, an alkali is added into an organic phase for neutralization, and after the liquid separation, the organic phase is desolventized to obtain 1-[2-chloro-3-(bromomethyl)-4-(methylsulfonyl)phenyl]ethanone as a solid. The above alkali for neutralization is not particularly limited, and for example, may be sodium hydroxide and/or potassium hydroxide or the like.

According to the present invention, preferably, the initiator is one or more of azobisisobutyronitrile, azobisisoheptonitrile, benzoyl peroxide and tert-butyl hydroperoxide; and more preferably, the initiator is azobisisobutyronitrile. According to the present invention, preferably, the initiator is used in an amount of 0.01-0.5 mol relative to 1 mol of 1-[2-chloro-3-(methyl)-4-(methylsulfonyl)phenyl]ethanone; more preferably, the initiator is used in an amount of 0.02-0.1 mol relative to 1 mol of 1-[2-chloro-3-(methyl)-4-(methylsulfonyl)phenyl]ethanone; and further preferably, the initiator is used in an amount of 0.03-0.06 mol relative to 1 mol of 1-[2-chloro-3-(methyl)-4-(methylsulfonyl)phenyl]ethanone. It is found through the research that if the amount of the initiator used is too low, a good initiation effect can not be achieved.

Preferably, in the above method, the metal tetrahydrofurfuryl alcoholate in the step (2) is sodium tetrahydrofurfuryl alcoholate or potassium tetrahydrofurfuryl alcoholate; and the reaction in the step (2) is carried out in a solvent A which is one or more of tetrahydrofuran, 2-methyltetrahydrofuran, 1,2-dichloroethane, chlorobenzene, dimethyl carbonate, ethylene glycol dimethyl ether and ethylene glycol diethyl ether; more preferably tetrahydrofuran, 2-methyltetrahydrofuran, ethylene glycol dimethyl ether and/or ethylene glycol diethyl ether; and particularly preferably tetrahydrofuran and/or 2-methyltetrahydrofuran. Preferably, the solvent A is used in an amount of 1000-3000 mL relative to 1 mol of 1-[2-chloro-3-(bromomethyl)-4-(methylsulfonyl)phenyl]ethanone; and more preferably, the solvent A is used in an amount of 1500-2500 mL relative to 1 mol of 1-[2-chloro-3-(bromomethyl)-4-(methylsulfonyl)phenyl]ethanone.

In a preferred embodiment of the present invention, the solvent A is the same as the solvent other than the water in the step (1).

Preferably, in the above method, sodium tetrahydrofurfuryl alcoholate and/or potassium tetrahydrofurfuryl alcoholate is used in an amount of 1-1.5 mol relative to 1 mol of 1-[2-chloro-3-(bromomethyl)-4-(methylsulfonyl)phenyl]ethanone; more preferably, sodium tetrahydrofurfuryl alcoholate and/or potassium tetrahydrofurfuryl alcoholate is used in an amount of 1-1.2 mol relative to 1 mol of 1-[2-chloro-3-(bromomethyl)-4-(methylsulfonyl)phenyl]ethanone; and further preferably, sodium tetrahydrofurfuryl alcoholate and/or potassium tetrahydrofurfuryl alcoholate is used in an amount of 1-1.1 mol relative to 1 mol of 1-[2-chloro-3-(bromomethyl)-4-(methylsulfonyl)phenyl]ethanone. It is found through the research that when the amount of sodium tetrahydrofurfuryl alcoholate and/or potassium tetrahydrofurfuryl alcoholate used is too high, an impurity in which a chlorine atom on a benzene ring is also substituted will appear in the reaction, and the impurity also increases as the amount of sodium tetrahydrofurfuryl alcoholate and/or potassium tetrahydrofurfuryl alcoholate used increases.

In the present invention, structural formulae of etherification of the bromobenzyl position, the disubstituted impurity in which a chlorine atom on the benzene ring is also substituted, and a hydrolysis impurity of bromobenzyl are as follows:

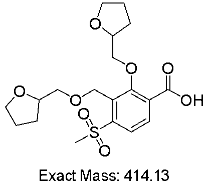

### Disubstituted impurity

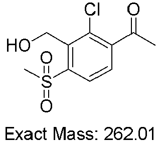

### Hydrolysis Impurity

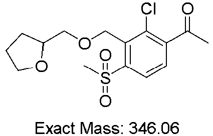

### Etherification of the bromobenzyl position (Formula 5).

In a preferred embodiment of the present invention, in the step (2), in a mixture of 1-[2-chloro-3-(bromomethyl)-4-(methylsulfonyl)phenyl]ethanone and a portion of a solvent, a mixture of sodium tetrahydrofurfuryl alcoholate and/or potassium tetrahydrofurfuryl alcoholate and another portion of the solvent is added dropwise. The another portion of the solvent is used such that the total content of sodium tetrahydrofurfuryl alcoholate and/or potassium tetrahydrofurfuryl alcoholate in the mixture of sodium tetrahydrofurfuryl alcoholate and/or potassium tetrahydrofurfuryl alcoholate and another portion of the solvent is 20-50% by weight, preferably 30-40% by weight.

In another preferred embodiment of the present invention, sodium tetrahydrofurfuryl alcoholate and/or potassium tetrahydrofurfuryl alcoholate is used in the form of tetrahydrofurfuryl alcohol as a solvent, and the content of sodium tetrahydrofurfuryl alcoholate and/or potassium tetrahydrofurfuryl alcoholate in a solution of sodium tetrahydrofurfuryl alcoholate and/or potassium tetrahydrofurfuryl alcoholate in tetrahydrofurfuryl alcohol may be 20-50% by weight, preferably 30-40% by weight.

According to the present invention, preferably, in the step (2), the conditions of the reaction include: a reaction temperature of -10°C to 100°C and a reaction time of 3-10 h; and more preferably, the conditions of the reaction include: a reaction temperature of 0-25°C and a reaction time of 3-8 h.

According to the present invention, after the reaction in the step (2) is finished, a target product can be obtained according to a conventional refining method in the art, and preferably, after the reaction in the step (2) is finished, only an insoluble matter needs to be filtered, and a filtrate is desolventized under a negative pressure to obtain 1-{2-chloro-3-[(*RS*-tetrahydrofuran-2-ylmethoxy)methyl]-4-(methylsulfonyl)phenyl}ethanone. In addition, in the case where water is used in the step (2) (that is, in the case where sodium tetrahydrofurfuryl alcoholate and/or potassium tetrahydrofurfuryl alcoholate are used in the form of a tetrahydrofurfuryl alcohol solution), after the reaction is completed, the resulting reaction solution is allowed to stand for layering, an organic phase is separated, and the organic phase is desolventized under a negative pressure to obtain 1-{2-chloro-3-[(*RS*-tetrahydrofuran-2-ylmethoxy)methyl]-4-(methylsulfonyl)phenyl}ethanone.

The filtrate and the organic phase can also be used directly in a reaction of a next step, and hence, the step (2) and the reaction of the next step, i.e., the step (3) can be carried out in the same solvent, and thus, the step (1), the step (2) and the step (3) can be carried out in the same solvent, which is very suitable for industrial preparation.

Preferably, in the above method, 1-{2-chloro-3-[(*RS*-tetrahydrofuran-2-ylmethoxy)methyl]-4-(methylsulfonyl)phenyl}ethanone is subjected to an oxidation reaction with an oxidizing agent to obtain 2-chloro-3-[(*RS*-tetrahydrofuran-2-ylmethoxy)methyl]-4-(methylsulfonyl)benzoic acid in the step (3). According to the present invention, preferably, the oxidation reaction in the step (3) is carried out in the presence of a catalyst, and the catalyst can adopt various catalysts generally used in the art for catalyzing the oxidation reaction, and for example, one or more of cobalt acetate, manganese acetate, copper acetate, vanadium pentoxide, selenium dioxide, ruthenium on carbon and ruthenium dioxide, preferably one or more of cobalt acetate, manganese acetate, selenium dioxide and ruthenium dioxide. In the present invention, preferably, the catalyst is used in an amount of 0.01-0.5 mol relative to 1 mol of 1-{2-chloro-3-[(*RS*-tetrahydrofuran-2-ylmethoxy)methyl]-4-(methylsulfonyl)phenyl}ethanone; more preferably, the catalyst is used in an amount of 0.02-0.3 mol relative to 1 mol of 1-{2-chloro-3-[(*RS*-tetrahydrofuran-2-ylmethoxy)methyl]-4-(methylsulfonyl)phenyl}ethanone; and preferably, the catalyst is used in an amount of 0.05-0.1 mol relative to 1 mol of 1-{2-chloro-3-[(*RS*-tetrahydrofuran-2-ylmethoxy)methyl]-4-(methylsulfonyl)phenyl}ethanone. According to the present invention, preferably, the reaction in the step (3) is carried out in a solvent B which is one or more of chlorobenzene, toluene, acetic acid, tert-butanol, isopropanol, dimethyl carbonate, ethylene glycol dimethyl ether, ethylene glycol diethyl ether, diethylene glycol dimethyl ether and pyridine; and more preferably, the solvent B is one or more of chlorobenzene, toluene, ethylene glycol dimethyl ether, ethylene glycol diethyl ether and diethylene glycol dimethyl ether. In the present invention, preferably, the solvent B is used in an amount of 1000-3000 mL relative to 1 mol of 1-{2-chloro-3-[(*RS*-tetrahydrofuran-2-ylmethoxy)methyl]-4-(methylsulfonyl)phenyl}ethanone; and more preferably, the solvent B is used in an amount of 1500-1800 mL relative to 1 mol of 1-{2-chloro-3-[(*RS-*tetrahydrofuran-2-ylmethoxy)methyl]-4-(methylsulfonyl)phenyl}ethanone.

In a preferred embodiment of the present invention, the solvent B is the same as the solvent A.

In another preferred embodiment of the present invention, the solvent B is the same as the solvent other than the water in the step (1).

In another preferred embodiment of the present invention, the solvent B is the same as the solvent A and the solvent other than the water in the step (1).

According to the present invention, preferably, the oxidizing agent may be various oxidizing agents commonly used in the art, and may be, for example, one or more of oxygen, compressed air, ozone, hydrogen peroxide, nitric acid, manganese dioxide, sodium periodate and potassium periodate.

In the present invention, preferably, the oxidizing agent is used in an amount conventional in the art according to the specifically selected oxidizing agent. For example, the oxidizing agent is used in an amount of 1-10 mol relative to 1 mol of 1-{2-chloro-3-[(*RS*-tetrahydrofuran-2-ylmethoxy)methyl]-4-(methylsulfonyl)phenyl}ethanone; and more preferably, the oxidizing agent is used in an amount of 1-5 mol relative to 1 mol of 1-{2-chloro-3-[(*RS*-tetrahydrofuran-2-ylmethoxy)methyl]-4-(methylsulfonyl)phenyl}ethanone.

According to the present invention, preferably, the conditions of the oxidation reaction include: a reaction temperature of 20-250°C and a reaction time of 0.5-10 h; and more preferably, the conditions of the oxidation reaction include: a reaction temperature of 20-120°C and a reaction time of 2-5 h. According to the present invention, after completion of the oxidation reaction, a target product can be obtained according to a conventional refining method in the art, and preferably, a part of the solvent is removed from the reaction product, and the temperature is reduced for crystallization to obtain the target product 2-chloro-3-[(*RS*-tetrahydrofuran-2-ylmethoxy)methyl]-4-(methylsulfonyl)benzoic acid. Preferably, in the above method of the present invention, 1-{2-chloro-3-[(*RS*-tetrahydrofuran-2-ylmethoxy)methyl]-4-(methylsulfonyl)phenyl}ethanone is subjected to a haloform reaction with a haloform reaction reagent, followed by acidification by addition of an aqueous solution of an acid to obtain 2-chloro-3-[(*RS*-tetrahydrofuran-2-ylmethoxy)methyl]-4-(methylsulfonyl)benzoic acid in the step (3). According to the present invention, preferably, the haloform reaction reagent is sodium hypochlorite, sodium hypobromite or liquid bromine+sodium hydroxide; and more preferably, the haloform reaction reagent is sodium hypochlorite.

According to the present invention, preferably, the haloform reaction in the above method is carried out in a solvent C which is one or more of 1,2-dichloroethane, chloroform, carbon tetrachloride and chlorobenzene; and more preferably, the solvent C is 1,2-dichloroethane. Preferably, the solvent C is used in an amount of 1000-3000 mL relative to 1 mol of 1-{2-chloro-3-[(*RS*-tetrahydrofuran-2-ylmethoxy)methyl]-4-(methylsulfonyl)phenyl}ethanone; and more preferably, the solvent C is used in an amount of 1500-1800 mL relative to 1 mol of 1-{2-chloro-3-[(*RS*-tetrahydrofuran-2-ylmethoxy)methyl]-4-(methylsulfonyl)phenyl}ethanone.

In the present invention, preferably, the haloform reaction reagent is used in an amount of 3-4.5 mol in terms of effective halogen relative to 1 mol of 1-{2-chloro-3-[(*RS*-tetrahydrofuran-2-ylmethoxy)methyl]-4-(methylsulfonyl)phenyl}ethanone; and more preferably, the haloform reaction reagent is used in an amount of 3-3.6 mol in terms of effective halogen relative to 1 mol of 1-{2-chloro-3-[(*RS*-tetrahydrofuran-2-ylmethoxy)methyl]-4-(methylsulfonyl)phenyl}ethanone.

According to the present invention, preferably, the conditions of the haloform reaction include: a reaction temperature of 20-80°C and a reaction time of 0.5-10 h; and more preferably, the conditions of the haloform reaction include: a reaction temperature of 20-50°C and a reaction time of 3-5 h.

According to the present invention, in the above preferred preparation method, the acid in the aqueous solution of the acid added after the end of the haloform reaction may be an inorganic acid and an organic acid, preferably, the acid is one or more of hydrochloric acid, sulfuric acid, acetic acid, formic acid and phosphoric acid; and preferably, the acid used for the acidification is hydrochloric acid and/or sulfuric acid. In addition, the acidification is preferably such that a pH value of a reaction system is 3.5 or less. Specifically, the pH value can be, for example, 3.5, 3.4, 3.3, 3.2, 3.1, 3, 2.9, 2.8, 2.6, 2.4, 2.2, 2, 1.8, 1.6, 1.4, 1.2, 1, 0.5 or the like, and is in the range formed by any two of the above values. According to the present invention, preferably, the acidification in the above preparation method is performed at a temperature of -30°C to 105°C; more preferably, the acidification is is performed at a temperature of 40-80°C; and further preferably, the acidification is performed at a temperature of 40-60°C. In a preferred embodiment of the present invention, the aqueous solution of the acid (e.g., sulfuric acid, hydrochloric acid, etc.) is added after the haloform reaction, so that the resulting reaction solution is neutralized to pH=6-7, the reaction solution is allowed to stand for liquid separation, an aqueous phase is separated and acidified at 40-50°C by addition of the aqueous solution of the acid to pH=3-3.3, stirring is then performed at 40-50°C for 1-5 h, acidification is performed at 40-50°C by addition of an aqueous solution of an acid to pH=1-2, and stirring is then performed at 40-50°C for 1-3 h.

According to the present invention, after the acidification is completed, a target product can be obtained according to a conventional refining method in the art, and preferably, the acidified product is subjected to solid-liquid separation to obtain the target product 2-chloro-3-[(*RS*-tetrahydrofuran-2-ylmethoxy)methyl]-4-(methylsulfonyl)benzoic acid.

Preferably, the present invention also provides a compound of a formula (5),

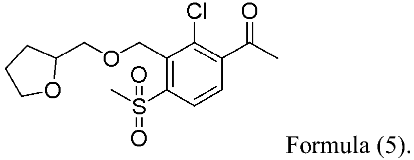

Preferably, the present invention also provides a compound of a formula (6),

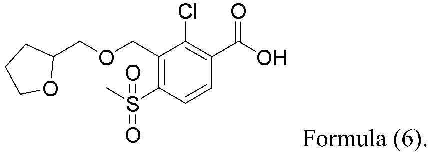

Particularly preferably, the present invention provides use of the compound of the formula (2) and the compound of the formula (5) in the preparation of 2-chloro-3-[(*RS*-tetrahydrofuran-2-ylmethoxy)methyl]-4-(methylsulfonyl)benzoic acid. In the present invention, by the use of the compound of the formula (2) and the compound of the formula (5) in the preparation of 2-chloro-3-[(*RS*-tetrahydrofuran-2-ylmethoxy)methyl]-4-(methylsulfonyl)benzoic acid, the process steps can be greatly shortened, the requirements of the reaction conditions are reduced, the process is more environmentally friendly, the production cost is reduced, and the reaction conditions are more suitable for industrialization.

Hereinafter, the present invention will be described in detail with reference to Examples, but the present invention is not limited to the following Examples, and raw materials in the Examples can be purchased unless otherwise specified, and the percentage in the Examples generally refers to a percentage by weight according to conventional understanding, and HPLC used in the present invention is Agilent 1260, and a chromatographic column is ZORBAX RX-C8.

### Example 1 Preparation of 2-chloro-3-(trifluoroethoxymethyl)-4-(methylsulfonyl)benzoic acid

### 1.1 Preparation of 1-[2-chloro-3-(bromomethyl)-4-(methylsulfonyl)phenyl]ethanone

A 2000 ml four-necked reaction flask equipped with mechanical stirring, a thermometer, a reflux device and a dropwise addition device was charged with 0.5 mol of 1-[2-chloro-3-(methyl)-4-(methylsulfonyl)phenyl]ethanone, 0.4 mol of sodium bromate, 0.2 mol of sodium bromide, 600 ml of chlorobenzene, and 125 ml of water were added, heating was performed with stirring until materials were fully dissolved, the temperature was raised to 78°C, 0.025 mol of AIBN (azobisisobutyronitrile) was charged, heat preservation was performed for 5 min, a 30% aqueous solution of H₂SO₄ (0.3 mol) was added dropwise at 78-83°C, after completion of the dropwise addition, heat preservation was performed at 80°C for 2 h, sampling was performed for in-process control by HPLC, after completion of the reaction, the resulting reaction solution was allowed to be stand for liquid separation, an organic phase was neutralized with sodium hydroxide to be neutral, after the liquid separation, the organic phase was desolventized until no more fraction was extracted under a vacuum degree of 0.095 MPa at 85°C, and cooling was performed to obtain a white solid (with a content of 98%, yield: 95%), which was determined by NMR and mass spectrometry to obtain an intermediate 1-[2-chloro-3-(bromomethyl)-4-(methylsulfonyl)phenyl]ethanone. NMR characterization data is as follows:
¹H-NMR (500 Hz, DMSO): δ 2.61 (d, 3H, J=54.5 Hz), δ 3.43 (t, 3H, J=15.5 Hz), δ 5.16 (S, 2H), δ 7.85 (d, 1H, J=8.5 Hz), δ 8.07 (d, 1H, J=8.5Hz).

### 1.2 Preparation of 1-[2-chloro-3-(trifluoroethoxymethyl)-4-(methylsulfonyl)phenyl]ethanone

50 g of the solid product in the step 1.1, i.e., 1-[2-chloro-3-(bromomethyl)-4-(methylsulfonyl)phenyl]ethanone (with a content of 98%, 0.151 mol) was completely dissolved in 200 ml of ethylene glycol dimethyl ether in a 500 ml four-necked reaction flask equipped with mechanical stirring, a thermometer, a reflux device and a dropwise addition device, the temperature was reduced to 10°C, 62.7 g of a solution of sodium trifluoroethoxide in ethylene glycol dimethyl ether (with a content of 30%, 0.154 mol) was added dropwise at 10-20°C, the dropwise addition being complete within 3 h, the temperature was kept at 20°C or below for 1 h, sampling was performed for in-process control by HPLC, after the reaction was complete, an insoluble material was filtered, a filtrate was desolventized until no more fraction was extracted under a vacuum degree of 0.095 MPa at 85°C, and cooling was performed to obtain an amber honey-like liquid, which was determined by NMR and mass spectrometry to obtian 53.64 g of an intermediate 1-[2-chloro-3-(trifluoroethoxymethyl)-4-(methylsulfonyl)phenyl]ethanone (with a content of 92%, yield: 95%). In addition to containing a hydrolysis impurity of bromobenzyl, a disubstituted impurity is also added (in addition to etherification of the bromobenzyl position, chlorine on a benzene ring is also substituted). NMR characterization data of 1-[2-chloro-3-(trifluoroethoxymethyl)-4-(methylsulfonyl)phenyl]ethanone is as follows:
¹H-NMR (500 Hz, DMSO): δ 2.61 (d, 3H, J=52.5 Hz), δ 3.35 (d, 3H, J=14 Hz), δ 4.29 (dd, 2H, J₁=9.5Hz, J₂=18.5Hz), δ 5.22 (S, 2H), δ 7.86 (d, 1H, J=8.5Hz), δ 8.07 (d, 1H, J=8.5Hz).

### 1.3 Preparation of 2-chloro-3-(trifluoroethoxymethyl)-4-(methylsulfonyl)benzoic acid

53.64 g of the product in the step 1.2, i.e., 1-[2-chloro-3-(trifluoroethoxymethyl)-4-(methylsulfonyl)phenyl]ethanone (with a content of 92%, 0.143 mol) was completely dissolved in 200 ml of 1,2-dichloroethane in a 1000 ml four-necked reaction flask equipped with mechanical stirring, a thermometer and a reflux device at room temperature to obtain an amber clear solution, 320.1 g of a sodium hypochlorite solution (10% of available chlorine, 0.43 mol) was added dropwise at 30-35°C, the dropwise addition being complete within 2 h, after the dropwise addition was complete, heat preservation was performed at 35°C for 2 h, sampling was performed for in-process control by HPLC, after the reaction was complete, heating was performed to 45°C, neutralization was performed by dropwise addition of 30 wt.% sulfuric acid to pH=6-7, the neutralized solution was allowed to stand for liquid separation, an aqueous phase was separated, followed by acidification by subsequent dropwise addition of 30 wt. % sulfuric acid at 45°C, turbidity occurred at pH=3.2, crystallization was performed under stirring at 45°C for 3 h while heat preservation, acidification was then performed by further addition of 30 wt.% sulfuric acid at 45°C to pH=1-2, followed by stirring for 1 h, filtration was performed to obtain an off-white solid, and the off-white solid was oven-dried, which was determined by NMR and mass spectrometry to obtain 46.95 g of 2-chloro-3-(trifluoroethoxymethyl)-4-(methylsulfonyl)benzoic acid (with a content of 98%, yield: 93%).

### Example 2 Preparation of 2-chloro-3-(trifluoroethoxymethyl)-4-(methylsulfonyl)benzoic acid

### 2.1 Preparation of 1-[2-chloro-3-(bromomethyl)-4-(methylsulfonyl)phenyl]ethanone

A 1000 ml four-necked reaction flask equipped with mechanical stirring, a thermometer, a reflux device and a dropwise addition device was charged with 0.5 mol of 1-[2-chloro-3-(methyl)-4-(methylsulfonyl)phenyl]ethanone, 0.4 mol of sodium bromate, 0.22 mol of sodium bromide, 500 ml of 1,2-dichloroethane, and 125 ml of water were added, heating was performed with stirring until materials were fully dissolved, the temperature was raised to 78°C, 0.025 mol of AIBN (azobisisobutyronitrile) was charged, heat preservation was performed for 5 min, a 30% aqueous solution of H₂SO₄ (0.3 mol) was added dropwise at 78-83°C, after completion of the dropwise addition, heat preservation was performed at 80°C for 2 h, sampling was performed for in-process control by HPLC, after completion of the reaction, the resulting reaction solution was allowed to be stand for liquid separation, an organic phase was neutralized with sodium hydroxide to be neutral, after the liquid separation, the organic phase was desolventized until no more fraction was extracted under a vacuum degree of 0.095 MPa at 85°C, and cooling was performed to obtain a white solid, which was determined by NMR and mass spectrometry to obtian an intermediate 1-[2-chloro-3-(bromomethyl)-4-(methylsulfonyl)phenyl]ethanone (with a content of 98%, yield: 94.8%).

### 2.2 Preparation of 1-[2-chloro-3-(trifluoroethoxymethyl)-4-(methylsulfonyl)phenyl]ethanone

50 g of the intermediate solid in the step 1) (with a content of 98%, 0.151 mol) was completely dissolved in 200 ml of chlorobenzene in a 500 ml four-necked reaction flask equipped with mechanical stirring, a thermometer, a reflux device and a dropwise addition device, the temperature was reduced to 10°C, an aqueous solution of sodium trifluoroethoxide (with a content of 30%, 0.154 mol) was added dropwise at 10-20°C, the dropwise addition being complete within 3 h, the temperature was kept at 20°C or below for 1 h, sampling was performed for in-process control by HPLC, after the reaction was complete, an aqueous phase was separated, and an organic phase was desolventized under a negative pressure until no more fraction was extracted under a vacuum degree of 0.095 MPa at 85°C, and cooling was performed to obtain an amber honey-like liquid, which was determined by NMR and mass spectrometry to obtain 52.94 g of an intermediate 1-[2-chloro-3-(trifluoroethoxymethyl)-4-(methylsulfonyl)phenyl]ethanone (with a content of 92%, yield: 93.77%). No hydrolysis impurity of bromobenzyl and disubstituted impurity were detected.

### 2.3 Preparation of 2-chloro-3-(trifluoroethoxymethyl)-4-(methylsulfonyl)benzoic acid

52.94 g of the product in the step 2.2, i.e., 1-[2-chloro-3-(trifluoroethoxymethyl)-4-(methylsulfonyl)phenyl]ethanone (with a content of 92%, 0.1415 mol) was completely dissolved in 200 ml of chlorobenzene in a 1000 ml four-necked reaction flask equipped with mechanical stirring, a thermometer and a reflux device, cooling was performed to 30°C to obtain an amber clear solution, 320.1 g of a sodium hypochlorite solution (10% of available chlorine, 0.43 mol) was added dropwise at 30-35°C, the dropwise addition being complete within 2 h, after the dropwise addition was complete, heat preservation was performed at 35°C for 2 h, sampling was performed for in-process control by HPLC, after the reaction was complete, heating was performed to 45°C, neutralization was performed by dropwise addition of 30 wt.% sulfuric acid to pH=6-7, the neutralized solution was allowed to stand for liquid separation, an aqueous phase was separated, followed by acidification by subsequent dropwise addition of 30 wt.% sulfuric acid at 45°C, turbidity occurred at pH=3.2, crystallization was performed under stirring at 45°C for 3 h while heat preservation, acidification was then performed by further addition of 30 wt.% sulfuric acid at 45°C to pH=1-2, followed by stirring for 1 h, filtration was performed to obtian an off-white solid, and the off-white solid was oven-dried, which was determined by NMR and mass spectrometry to obtain 45.26 g of 2-chloro-3-(trifluoroethoxymethyl)-4-(methylsulfonyl)benzoic acid (with a content of 98%, yield: 90.6%).

### Example 3 Preparation of 2-chloro-3-(trifluoroethoxymethyl)-4-(methylsulfonyl)benzoic acid

### 3.1 Preparation of 1-[2-chloro-3-(bromomethyl)-4-(methylsulfonyl)phenyl]ethanone

A 2000 ml four-necked reaction flask equipped with mechanical stirring, a thermometer, a reflux device and a dropwise addition device was charged with 0.5 mol of 1-[2-chloro-3-(methyl)-4-(methylsulfonyl)phenyl]ethanone, 0.2 mol of sodium bromate, 0.39 mol of sodium bromide, 1000ml of chlorobenzene, and 125ml of water were added, heating was performed with stirring until materials were fully dissolved, after heating was performed to 70°C, 0.025 mol of AIBN (azobisisobutyronitrile) was charged, heat preservation was performed for 5 min, 0.3 mol of a 30% aqueous H₂SO₄ solution was added dropwise at 70-75°C, after completion of the dropwise addition, heat preservation was performed at 70°C for 4 h, sampling was performed for in-process control by HPLC, and after completion of the reaction, the resulting reaction solution was allowed to be stand for liquid separation to obtain 1274.6 g of a solution of an intermediate 1-[2-chloro-3-(bromomethyl)-4-(methylsulfonyl)phenyl]ethanone (with a content of 12.1%, yield: 95.2%).

### 3.2 Preparation of 1-[2-chloro-3-(trifluoroethoxymethyl)-4-(methylsulfonyl)phenyl]ethanone

404.4 g of the solution of 1-[2-chloro-3-(bromomethyl)-4-(methylsulfonyl)phenyl]ethanone (with a content of 12.1%, 0.151 mol) in the step 3.1 was heated to 40°C in a 500 ml four-necked reaction flask equipped with mechanical stirring, a thermometer, a reflux device and a dropwise addition device, an aqueous solution of sodium trifluoroethoxide (with a content of 30%, 0.154 mol) was added dropwise at 40-45°C, the dropwise addition being complete within 3 h, the temperature was kept at 40°C or below for 30 min, sampling was performed in-process control by HPLC, and after the reaction was complete, an aqueous phase was separated to obtain 376.7 g of a solution of an intermediate 1-[2-chloro-3-(trifluoroethoxymethyl)-4-(methylsulfonyl)phenyl]ethanone in chlorobenzene (with a content of 13.1%, yield 95%). A hydrolysis impurity of bromobenzyl and a disubstituted impurity were not detected.

### 3.3 Preparation of 2-chloro-3-(trifluoroethoxymethyl)-4-(methylsulfonyl)benzoic acid

The solution of 1-[2-chloro-3-(trifluoroethoxymethyl)-4-(methylsulfonyl)phenyl]ethanone in chlorobenzene (with a content of 13.1%, 0.143 mol) in the step 3.2 was added to a 1000 ml four-necked flask equipped with mechanical stirring, a thermometer, and a reflux device, stirring and heating were performed, 320.1 g of a sodium hypochlorite solution (10% of available chlorine, 0.43 mol) was added dropwise at 30-35°C, the dropwise addition being complete within 2 h, after the dropwise addition was complete, heat preservation was performed at 35°C for 2 h, sampling was performed for in-process control by HPLC, after the reaction was complete, heating was performed to 45°C, neutralization was performed by dropwise addition of 30 wt.% sulfuric acid to pH=6-7, the neutralized solution was allowed to stand for liquid separation, an aqueous phase was separated, followed by acidification by subsequent dropwise addition of 30 wt.% sulfuric acid at 45°C, turbidity occurred at pH=3.2, crystallization was performed under stirring at 45°C for 3 h while heat preservation, acidification was then performed by further addition of 30 wt.% sulfuric acid at 45°C to pH=1-2, followed by stirring for 1 h, filtration was performed to obtian an off-white solid, and the off-white solid was oven-dried, which was determined by NMR and mass spectrometry to obtian 47.76 g of 2-chloro-3-(trifluoroethoxymethyl)-4-(methylsulfonyl)benzoic acid (with a content of 98%, yield: 94.6%).

### Example 4 Preparation of 2-chloro-3-(trifluoroethoxymethyl)-4-(methylsulfonyl)benzoic acid

53.3 g of 1-[2-chloro-3-(trifluoroethoxymethyl)-4-(methylsulfonyl)phenyl]ethanone (with a content of 92%, 0.1425 mol) prepared according to the steps 1.1 and 1.2 of Example 1 was dissolved completely with 200 ml of acetic acid at room temperature, manganese acetate (with a content of 98%, 0.014 mol) was added, heating was performed to 120°C, O₂ was introduced until a pressure was 0.3 MPa, the temperature was kept for 3 h at this pressure, sampling was performed for in-process control by HPLC, after the reaction was complete, acetic acid was removed until no fraction at 100°C under a vacuum degree of 0.097 MPa, the temperature was slightly reduced to 90°C, 200 ml of water was added, stirring was performed for 1 h, filtration was performed to obtian an off-white solid, and the off-white solid was oven-dried, which was determined by NMR and mass spectrometry to obtain 46.03 g of 2-chloro-3-(trifluoroethoxymethyl)-4-(methylsulfonyl)benzoic acid (with a content of 98%, yield: 91.5%).

### Example 5 Preparation of 2-chloro-3-(trifluoroethoxymethyl)-4-(methylsulfonyl)benzoic acid

55.5 g of 1-[2-chloro-3-(trifluoroethoxymethyl)-4-(methylsulfonyl)phenyl]ethanone (with a content of 90%, 0.1452 mol) prepared according to the steps 2.1 and 2.2 of Example 2 was dissolved completely with 250 ml of tert-butanol, selenium dioxide (with a content of content 98%, 0.0029 mol) was added, hydrogen peroxide (with a content of 30%, 0.1525 mol) was added dropwise at room temperature, the dropwise addition being complete at 20-25°C within 4 h, heat preservation was performed for 3 h, sampling was performed for in-process control by HPLC, after the reaction was complete, little sodium bisulfite was added, tert-butanol was removed at atmospheric pressure until no fraction at 100°C, the temperature was reduced slightly to 90°C, 200 ml of water was added, stirring was performed for 1 h, filtration was performed to obtain an off-white solid, and the off-white solid was oven-dried, which was determined by NMR and mass spectrometry to obtain 47 g of 2-chloro-3-(trifluoroethoxymethyl)-4-(methylsulfonyl)benzoic acid (with a content of 96%, yield: 89.81%).

### Example 6 Preparation of 1-[2-chloro-3-(bromomethyl)-4-(methylsulfonyl)phenyl]ethanone

Following the steps of Example 1, a bromine source was replaced with a combination of hydrobromic acid and hydrogen peroxide, wherein in a 2000 ml four-necked flask equipped with mechanical stirring, a thermometer, and a reflux device, 0.5 mol of 1-[2-chloro-3-(methyl)-4-(methylsulfonyl)phenyl]ethanone was added into 600 ml of chlorobenzene, 0.6 mol of 40% hydrobromic acid was then added, heating was performed to 78°C with stirring, 0.025 mol of AIBN (azobisisobutyronitrile) was charged, heat preservation was performed for 5 min, a 34% aqueous solution of H₂O₂ (containing 0.6 mol of H₂O₂) was added dropwise at 78-83°C, the dropwise addition being complete within about 5 h, after the the dropwise addition was completed, the temperature was kept at 80°C for 2 h, sampling was performed for in-process control by HPLC, after the reaction was completed, the resulting reaction solution was allowed to stand for liquid separation, excess hydrogen peroxide was neutralized by the addition of sodium bisulfite to an organic phase, neutralization was then performed by the addition of sodium hydroxide to be neutral, after the liquid separation, the organic phase was desolventized until no more fraction was extracted under a vacuum degree of 0.095 MPa at 85°C, and cooling was performed to obtian 152.8 g of 1-[2-chloro-3-(bromomethyl)-4-(methylsulfonyl)phenyl]ethanone (with a content of 88%, yield: 83%) as a white solid.

### Example 7 Preparation of 1-[2-chloro-3-(bromomethyl)-4-(methylsulfonyl)phenyl]ethanone

Following the steps of Example 1, a bromine source was replaced with a combination of hydrobromic acid and an aqueous sodium bromate solution, wherein in a 2000 ml four-necked flask equipped with mechanical stirring, a thermometer, and a reflux device, 0.5 mol of 1-[2-chloro-3-(methyl)-4-(methylsulfonyl)phenyl]ethanone was added into 600 ml of chlorobenzene, 0.6 mol of 40% hydrobromic acid was then added, heating was performed to 78°C with stirring, 0.025 mol of AIBN (azobisisobutyronitrile) was charged, heat preservation was performed for 5 min, a 30% aqueous solution of sodium bromate (0.6 mol) was added dropwise at 78-83°C, the dropwise addition being complete within 4 h, after the the dropwise addition was completed, the temperature was kept at 80°C for 2 h, sampling was performed for in-process control by HPLC, after the reaction was completed, the resulting reaction solution was allowed to stand for liquid separation, an organic phase was neutralized by addition of sodium hydroxide to be neutral, after the liquid separation, the organic phase was desolventized until no more fraction was extracted under a vacuum degree of 0.095 MPa at 85°C, and cooling was performed to obtain 151.08 g of 1-[2-chloro-3-(bromomethyl)-4-(methylsulfonyl)phenyl]ethanone (with a content of 89%, yield: 83%) as a white solid.

### Example 8 Preparation of 1-[2-chloro-3-(bromomethyl)-4-(methylsulfonyl)phenyl]ethanone

Following the steps of Example 1, a bromine source was replaced with DBH, wherein in a 1000 ml four-necked flask equipped with mechanical stirring, a thermometer, and a reflux device, 0.5 mol of 1-[2-chloro-3-(methyl)-4-(methylsulfonyl)phenyl]ethanone was added into 600 ml of chlorobenzene, heating was performed to 78°C with stirring, 0.025 mol of AIBN (azobisisobutyronitrile) was charged, heat preservation was performed for 5 min, solid DBH (0.3 mol) was uniformly added with a solid feeder at 78-83°C, the addition being completed within 4.5 h, heat preservation was performed at 80°C for 2 h, sampling was performed for in-process control by HPLC, after the reaction was completed, an insoluble matter was filtered, a filtrate was neutralized by addition of a 8% aqueous sodium bicarbonate solution to be neutral, after liquid separation, an organic phase was desolventized until no more fraction was extracted under a vacuum degree of 0.095 MPa at 85°C, and cooling was performed to obtian 148.06 g of 1-[2-chloro-3-(bromomethyl)-4-(methylsulfonyl)phenyl]ethanone (with a content of 93%, yield: 85%) as a white solid.

### Example 9 Preparation of 1-[2-chloro-3-(bromomethyl)-4-(methylsulfonyl)phenyl]ethanone

Following the steps of Example 1, a bromine source was replaced with NBS, wherein in a 1000 ml four-necked flask equipped with mechanical stirring, a thermometer, and a reflux device, 0.5 mol of 1-[2-chloro-3-(methyl)-4-(methylsulfonyl)phenyl]ethanone was added into 600 ml of chlorobenzene, heating was performed to 78°C with stirring, 0.025 mol of AIBN (azobisisobutyronitrile) was charged, heat preservation was performed for 5 min, solid NBS (0.6 mol) was uniformly added with a solid feeder at 78-83°C, the addition being completed within 4.5 h, heat preservation was performed at 80°C for 2 h, sampling was performed for in-process control by HPLC, after the reaction was completed, water was added until a solid was completely dissolved, neutralization was performed by addition of a 8 wt% aqueous sodium bicarbonate solution to be neutral, after liquid separation, an organic phase was desolventized until no more fraction was extracted under a vacuum degree of 0.095 MPa at 85°C, and cooling was performed to obtain 153.29 g of 1-[2-chloro-3-(bromomethyl)-4-(methylsulfonyl)phenyl]ethanone (with a content of 93%, yield: 88%) as a white solid.

### Example 10 Preparation of 1-[2-chloro-3-(bromomethyl)-4-(methylsulfonyl)phenyl]ethanone

A 2000 ml four-necked reaction flask equipped with mechanical stirring, a thermometer, a reflux device and a dropwise addition device was charged with 0.5 mol of 1-[2-chloro-3-(methyl)-4-(methylsulfonyl)phenyl]ethanone, 0.4 mol of sodium bromate, 0.2 mol of sodium bromide, 600 ml of chlorobenzene, and 125 ml of water were added, heating was performed with stirring until materials were fully dissolved, the temperature was raised to 78°C, 0.0004 mol of AIBN (azobisisobutyronitrile) was charged, heat preservation was performed for 5 min, a 30 wt% aqueous solution of H₂SO₄ (0.3 mol) was added dropwise at 78-83°C, after completion of the dropwise addition, heat preservation was performed at 80°C for 2 h, sampling was performed for in-process control by HPLC, a conversion being 82%, after supplementary addition of AIBN to 0.0005 mol, heat preservation was then performed for 2 h, after conversion was complete by in-process control, the resulting reaction solution was allowed to be stand for liquid separation, an organic phase was neutralized with sodium hydroxide to be neutral, after the liquid separation, the organic phase was desolventized until no more fraction was extracted under a vacuum degree of 0.095 MPa at 85°C, and cooling was performed to obtain a white solid, which was determined by NMR and mass spectrometry to give 157.49 g of an intermediate 1-[2-chloro-3-(bromomethyl)-4-(methylsulfonyl)phenyl]ethanone (with a content of 97%, yield: 94.3%).

### Example 11 Preparation of 1-[2-chloro-3-(bromomethyl)-4-(methylsulfonyl)phenyl]ethanone

A 2000 ml four-necked reaction flask equipped with mechanical stirring, a thermometer, a reflux device and a dropwise addition device was charged with 0.5 mol of 1-[2-chloro-3-(methyl)-4-(methylsulfonyl)phenyl]ethanone, 0.4 mol of sodium bromate, 0.2 mol of sodium bromide, 600 ml of chlorobenzene, and 125 ml of water were added, heating was performed with stirring until materials were fully dissolved, the temperature was raised to 78°C, 0.26 mol of AIBN (azobisisobutyronitrile) was charged, heat preservation was performed for 5 min, a 30% aqueous solution of H₂SO₄ (0.3 mol) was added dropwise at 78-83°C, after completion of the dropwise addition, heat preservation was performed at 80°C for 2 h, sampling was performed for in-process control by HPLC, after conversion was complete by the in-process control, the resulting reaction solution was allowed to be stand for liquid separation, an organic phase was neutralized with sodium hydroxide to be neutral, after the liquid separation, the organic phase was desolventized until no more fraction was extracted under a vacuum degree of 0.095 MPa at 85°C, and cooling was performed to obtain a white solid, which was determined by NMR and mass spectrometry to obtain 153.29g of an intermediate 1-[2-chloro-3-(bromomethyl)-4-(methylsulfonyl)phenyl]ethanone (with a content of 93%, yield: 88%).

### Example12 Preparation of 1-[2-chloro-3-(trifluoroethoxymethyl)-4-(methylsulfonyl)phenyl]ethanone

After preparation of 1-[2-chloro-3-(bromomethyl)-4-(methylsulfonyl)phenyl]ethanone following the method of Example 1, 50 g of a solid 1-[2-chloro-3-(bromomethyl)-4-(methylsulfonyl)phenyl]ethanone (with a content of 98%, 0.151 mol) was completely dissolved in 200 ml of ethylene glycol dimethyl ether in a 1000 ml four-necked reaction flask equipped with mechanical stirring, a thermometer, a reflux device and a dropwise addition device, and the temperature was reduced to 10°C, 98.25 g of a solution of sodium trifluoroethoxide in ethylene glycol dimethyl ether (with a content of 30%, 0.242 mol) was added dropwise at 10-20°C, the dropwise addition being complete within 5 h, the temperature was kept at 20°C or below for 1 h, after sampling was performed for in-process control by HPLC, the reaction was complete but a disubstituted impurity (in addition to etherification of the bromobenzyl position, chlorine on a benzene ring was also substituted (8.6%, normalized after in-process control)) was also newly added in addition to a hydrolysis impurity of partial bromobenzyl (1.3%, normalized after in-process control), an insoluble material was filtered, a filtrate was desolventized under a negative pressure until no more fraction was extracted under a vacuum degree of 0.095 MPa at 85°C, and cooling was performed to obtain an amber honey-like liquid, and 49.58 g of an intermediate 1-[2-chloro-3-(trifluoroethoxymethyl)-4-(methylsulfonyl)phenyl]ethanone (with a content of 88%, yield: 84%) was obtained.

### Example 13 1-[2-chloro-3-(trifluoroethoxymethyl)-4-(methylsulfonyl)phenyl]ethanone

After preparation of 1-[2-chloro-3-(bromomethyl)-4-(methylsulfonyl)phenyl]ethanone following the method of Example 1, 50 g of a solid 1-[2-chloro-3-(bromomethyl)-4-(methylsulfonyl)phenyl]ethanone (with a content of 98%, 0.151 mol) was completely dissolved in 200 ml of ethylene glycol dimethyl ether in a 1000 ml four-necked reaction flask equipped with mechanical stirring, a thermometer, a reflux device and a dropwise addition device, and the temperature was reduced to 10°C. 58.34 g of an aqueous solution of sodium trifluoroethoxide (with a content of 30 wt%, 0.143 mol) was added dropwise at 10-20°C, the dropwise addition being complete within 5 h, the temperature was kept at 20°C or below for 1 h, and sampling was performed for in-process control by HPLC, wherein 9% of the raw materials remained. The aqueous solution of sodium trifluoroethoxide (30% by weight, 0.13 mol) was supplementarily added, the temperature was kept for 1 h after the completion of the dropwise addition, after the reaction was complete by in-process control, no hydrolysis impurity of bromobenzyl and disubstituted impurity were detected, an insoluble matter was filtered, a filtrate was desolventized under a negative pressure until no more fraction was extracted under a vacuum degree of 0.095 MPa at 85°C, and cooling was performed to obtain an amber honey-like liquid, and 46.92 g of, an intermediate 1-[2-chloro-3-(trifluoroethoxymethyl)-4-(methylsulfonyl)phenyl]ethanone (with a content of 93%, yield: 84%) was obtained.

### Example 14 Preparation of 2-chloro-3-[(RS-tetrahydrofuran-2-ylmethoxy)methyl]-4-(methylsulfonyl)benzoic acid

### 14.1 Preparation of 1-{2-chloro-3-[(RS-tetrahydrofuran-2-ylmethoxy)methyl]-4-(methylsulfonyl)phenyl}ethanone

After preparation of 1-[2-chloro-3-(bromomethyl)-4-(methylsulfonyl)phenyl]ethanone following the method of Example 1, 50 g of a solid 1-[2-chloro-3-(bromomethyl)-4-(methylsulfonyl)phenyl]ethanone (with a content of 98%, 0.151 mol) was completely dissolved in 200 ml of tetrahydrofuran in a 1000 ml four-necked reaction flask equipped with mechanical stirring, a thermometer, a reflux device and a dropwise addition device, heating was performed to 30°C, 82.46 g of a solution of sodium tetrahydrofurfuryl alcoholate in tetrahydrofuran (with a content of 25%, 0.166 mol) was added dropwise at 30-40°C, the dropwise addition being complete within about 3 h, after the dropwise addition was complete, the temperature was kept at 40°C for 3 h, wherein 0.5% of the raw materials remained, the content of a disubstituted impurity was 1.2%, and the content of a hydrolysis impurity was less than 0.1% after in-process control, an insoluble material was filtered, a filtrate was desolventized under a negative pressure until no more fraction was extracted under a vacuum degree of 0.095 MPa at 65°C, and cooling was performed to obtain a dark red honey-like liquid, which was determined as 51.11 g of an intermediate 1-{2-chloro-3-[(*RS*-tetrahydrofuran-2-ylmethoxy)methyl]-4-(methylsulfonyl)phenyl}ethanone (with a content of 92%, yield: 90%) by NMR and mass spectrometry. NMR characterization data of 1-{2-chloro-3-[(*RS*-tetrahydrofuran-2-ylmethoxy)methyl]-4-(methylsulfonyl)phenyl}ethanone is as follows:
¹H-NMR (500 Hz, DMSO): δ1.51~1.93 (m, 4H), δ2.60 (d, 3H, J=49 Hz), δ3.38 (d, 3H, J=28.5 Hz), δ3.55 (m, 2H), δ3.62, 3.73 (m, 2H), δ3.99 (m, 1H), δ5.06 (dd, 2H, J₁=11 Hz, J₂=12 Hz), δ7.82 (d, 1H, J=8.5 Hz), δ8.06 (d, 1H, J=8.5 Hz).

### 14.2 Preparation of 2-chloro-3-[(RS-tetrahydrofuran-2-ylmethoxy)methyl]-4-(methylsulfonyl)benzoic acid

51.11 g of the product in the step 14.1 (with a content of 92%, 0.136 mol) was completely dissolved in 200 ml of 1,2-dichloroethane and transferred to a 1000 ml four-necked reaction flask equipped with mechanical stirring, a thermometer, and a reflux condenser, heating was performed with stirring to 35°C, 313.4 g of an aqueous sodium hypochlorite solution (10% of available chlorine, 0.421 mol) was slowly added dropwise at 35-40°C, the dropwise addition being complete within 3 h, after the dropwise addition was complete, heat preservation was performed at 40°C for 2 h, sampling was performed for in-process control, after the reaction was complete, heating was performed to 45°C, neutralization was performed by dropwise addition of 30 wt.% sulfuric acid to pH=6-7, the neutralized solution was allowed to stand for liquid separation, an aqueous phase was separated, followed by acidification by subsequent dropwise addition of 30 wt.% sulfuric acid at 45°C, turbidity occurred at pH=3.2, crystallization was performed under stirring at 45°C for 3 h while heat preservation, acidification was then performed by further addition of 30 wt.% sulfuric acid at 45°C to pH=1-2, followed by stirring for 1 h, filtration was performed to obtain an off-white solid, and the off-white solid was oven-dried to obtain 43.88 g of 2-chloro-3-[(*RS*-tetrahydrofuran-2-ylmethoxy)methyl]-4-(methylsulfonyl)benzoic acid (with a content of 96%, yield: 89%).

### Example 15 Preparation of 1-{2-chloro-3-[(RS-tetrahydrofuran-2-ylmethoxy)methyl]-4-(methylsulfonyl)phenyl}ethanone

After preparation of 1-[2-chloro-3-(bromomethyl)-4-(methylsulfonyl)phenyl]ethanone following the method of Example 1, 50 g of a solid 1-[2-chloro-3-(bromomethyl)-4-(methylsulfonyl)phenyl]ethanone (with a content of 98%, 0.151 mol) was completely dissolved in 200 ml of 1,2-dichloroethane in a 1000 ml four-necked reaction flask equipped with mechanical stirring, a thermometer, a reflux device and a dropwise addition device, 10 ml of water was then added, heating was performed to 30°C, 82.46 g of a solution of sodium tetrahydrofurfuryl alcoholate in tetrahydrofurfuryl alcohol (with a content of 25%, 0.166 mol) was added dropwise at 30-40°C, the dropwise addition being complete within about 3 h, after the dropwise addition was complete, the temperature was kept at 40°C for 3 h, after in-process control, the reaction was complete, and a hydrolysis impurity and a disubstituted impurity were not generated, 100 ml of water was added to disslove an inorganic salt, liquid separation was performed, and an organic phase was desolventized until no more fraction was extracted under a vacuum degree of 0.095 MPa at 80°C, and cooling was performed to obtain a dark red honey-like liquid, which was determined as 51.7 g of an intermediate 1-{2-chloro-3-[(*RS*-tetrahydrofuran-2-ylmethoxy)methyl]-4-(methylsulfonyl)phenyl}ethanone (with a content of 95%, yield: 94%) by NMR and mass spectrometry.

Preferred embodiments of the present invention are described in detail above, however, the present invention is not limited thereto. Within the technical concept range of the present invention, the technical solution of the present invention can be subjected to various simple variations, including combinations of various technical features in any other suitable manner, and these simple variations and combinations should likewise be considered as the contents disclosed by the present invention, and all fall within the protection scope of the present invention.

## Claims

1. A preparation method for 2-chloro-3-(trifluoroethoxymethyl)-4-(methylsulfonyl)benzoic acid, **characterized in** comprising:
Step (1), subjecting 1-[2-chloro-3-(methyl)-4-(methylsulfonyl)phenyl]ethanone to a bromination reaction with a brominating agent in the presence of an initiator to obtain 1-[2-chloro-3-(bromomethyl)-4-(methylsulfonyl)phenyl]ethanone;
Step (2), reacting 1-[2-chloro-3-(bromomethyl)-4-(methylsulfonyl)phenyl]ethanone with metal trifluoroethoxide to obtain 1-[2-chloro-3-(trifluoroethoxymethyl)-4-(methylsulfonyl)phenyl]ethanone; and
Step (3), preparing 2-chloro-3-(trifluoroethoxymethyl)-4-(methylsulfonyl)benzoic acid from 1-[2-chloro-3-(trifluoroethoxymethyl)-4-(methylsulfonyl)phenyl]ethanone.

2. The method according to claim 1, **characterized in that** the reaction in the step (1) is carried out in a solvent selected from one or more of water, 1,2-dichloroethane, chlorobenzene, acetic acid, dimethyl carbonate, ethylene glycol dimethyl ether and ethylene glycol diethyl ether, preferably a combination of water and other solvents, more preferably one of a combination of chlorobenzene and water and a combination of 1,2-dichloroethane and water.

3. The method according to claim 1 or 2, **characterized in that** the brominating agent in the step (1) is selected from one or more of a combination of sodium bromate, sodium bromide and an acid, a combination of hydrogen peroxide and sodium bromide and an acid, a combination of hydrogen peroxide and hydrobromic acid, a combination of sodium bromate and hydrobromic acid, liquid bromine, NBS and DBH, preferably the combination of sodium bromate, sodium bromide and an acid, wherein the acid in the combination of sodium bromate, sodium bromide and an acid and the combination of hydrogen peroxide and sodium bromide and an acid is sulfuric acid and/or phosphoric acid;
and/or the initiator is one or more of azobisisobutyronitrile, azobisisoheptonitrile, benzoyl peroxide and tert-butyl hydroperoxide.

4. The method according to claim 3, **characterized in that** the water is used in an amount of 100-1500 mL relative to 1 mol of 1-[2-chloro-3-(methyl)-4-(methylsulfonyl)phenyl]ethenone in the step (1).

5. The method according to claim 4, **characterized in that** the initiator is used in an amount of 0.01-0.5 mol relative to 1 mol of 1-[2-chloro-3-(methyl)-4-(methylsulfonyl)phenyl]ethenone in the step (1).

6. The method according to claim 5, **characterized in that** the metal trifluoroethoxide in the step (2) is sodium trifluoroethoxide or potassium trifluoroethoxide; and the reaction in the step (2) is carried out in a solvent A which is one or more of tetrahydrofuran, 2-methyltetrahydrofuran, 1,2-dichloroethane, chlorobenzene, dimethyl carbonate, ethylene glycol dimethyl ether and ethylene glycol diethyl ether.

7. The method according to claim 6, **characterized in that** sodium trifluoroethoxide and/or potassium trifluoroethoxide is used in an amount of 1-1.5 mol relative to 1 mol of 1-[2-chloro-3-(bromomethyl)-4-(methylsulfonyl)phenyl]ethanone.

8. The method according to claim 7, **characterized in that** in the step (3), 1-[2-chloro-3-(trifluoroethoxymethyl)-4-(methylsulfonyl)phenyl]ethanone is subjected to an oxidation reaction with an oxidizing agent to obtain 2-chloro-3-(trifluoroethoxymethyl)-4-(methylsulfonyl)benzoic acid.

9. The method according to claim 8, **characterized in that** the oxidation reaction is carried out in the presence of a catalyst; the catalyst is one or more of cobalt acetate, manganese acetate, copper acetate, vanadium pentoxide, selenium dioxide, ruthenium on carbon, and ruthenium dioxide; and the oxidizing agent is one or more of oxygen, compressed air, ozone, hydrogen peroxide, nitric acid, manganese dioxide, sodium periodate, and potassium periodate.

10. The method according to claim 9, **characterized in that** the catalyst is used in an amount of 0.001-0.5 mol relative to 1 mol of 1-[2-chloro-3-(trifluoroethoxymethyl)-4-(methylsulfonyl)phenyl]ethanone; and the oxidizing agent is used in an amount of 1-10 mol, preferably 1-5 mol, relative to 1 mol of 1-[2-chloro-3-(trifluoroethoxymethyl)-4-(methylsulfonyl)phenyl]ethanone.

11. The method according to any one of claims 1-7, **characterized in that** in the step (3), 1-[2-chloro-3-(trifluoroethoxymethyl)-4-(methylsulfonyl)phenyl]ethanone is subjected to a haloform reaction with a haloform reaction reagent, followed by acidification by addition of an aqueous solution of an acid to obtain 2-chloro-3-(trifluoroethoxymethyl)-4-(methylsulfonyl)benzoic acid.

12. The method according to claim 11, **characterized in that** the haloform reaction reagent is one or more of a combination of sodium hypochlorite, sodium hypobromite, liquid bromine and sodium hydroxide; and the haloform reaction reagent is used in an amount of 3-4.5 mol in terms of effective halogen relative to 1 mol of 1-[2-chloro-3-(trifluoroethoxymethyl)-4-(methylsulfonyl)phenyl]ethanone.

13. The method according to claim 11 or 12, **characterized in that** in the step (3), the acid is one or more of hydrochloric acid, sulfuric acid, acetic acid, formic acid and phosphoric acid.

14. A preparation method for 2-chloro-3-[(*RS*-tetrahydrofuran-2-ylmethoxy)methyl]-4-(methylsulfonyl)benzoic acid, **characterized in** comprising:
Step (1), subjecting 1-[2-chloro-3-(methyl)-4-(methylsulfonyl)phenyl]ethanone to a bromination reaction with a brominating agent in the presence of an initiator to obtain 1-[2-chloro-3-(bromomethyl)-4-(methylsulfonyl)phenyl]ethanone;
Step (2), reacting 1-[2-chloro-3-(bromomethyl)-4-(methylsulfonyl)phenyl]ethanone with metal tetrahydrofurfuryl alcoholate to obtain 1-{2-chloro-3-[(*RS*-tetrahydrofuran-2-ylmethoxy)methyl]-4-(methylsulfonyl)phenyl}ethanone; and
Step (3), preparing 2-chloro-3-[(*RS*-tetrahydrofuran-2-ylmethoxy)methyl]-4-(methylsulfonyl)benzoic acid from 1-{2-chloro-3-[(*RS*-tetrahydrofuran-2-ylmethoxy)methyl]-4-(methylsulfonyl)phenyl}ethanone.

15. The method according to claim 14, **characterized in that** the brominating agent in the step (1) is selected from one or more of a combination of sodium bromate, sodium bromide and an acid, a combination of hydrogen peroxide and sodium bromide and an acid, a combination of hydrogen peroxide and hydrobromic acid, a combination of sodium bromate and hydrobromic acid, liquid bromine, NBS and DBH, preferably the combination of sodium bromate, sodium bromide and an acid, wherein the acid in the combination of sodium bromate, sodium bromide and an acid and the combination of hydrogen peroxide and sodium bromide and an acid is sulfuric acid and/or phosphoric acid;
and/or the initiator is one or more of azobisisobutyronitrile, azobisisoheptonitrile, benzoyl peroxide and tert-butyl hydroperoxide.

16. The method according to claim 14 or 15, **characterized in that** water is used in an amount of 100-1500 mL relative to 1 mol of 1-[2-chloro-3-(methyl)-4-(methylsulfonyl)phenyl]ethenone in the step (1).

17. The method according to claim 16, **characterized in that** the initiator is used in an amount of 0.01-0.5 mol relative to 1 mol of 1-[2-chloro-3-(methyl)-4-(methylsulfonyl)phenyl]ethenone in the step (1).

18. The method according to claim 17, **characterized in that** the metal tetrahydrofurfuryl alcoholate in the step (2) is sodium tetrahydrofurfuryl alcoholate or potassium tetrahydrofurfuryl alcoholate; and the reaction in the step (2) is carried out in a solvent A selected from one or more of tetrahydrofuran, 2-methyltetrahydrofuran, 1,2-dichloroethane, chlorobenzene, dimethyl carbonate, ethylene glycol dimethyl ether and ethylene glycol diethyl ether.

19. The method according to claim 18, **characterized in that** sodium tetrahydrofurfuryl alcoholate or potassium tetrahydrofurfuryl alcoholate is used in an amount of 1-1.5 mol relative to 1 mol of 1-[2-chloro-3-(bromomethyl)-4-(methylsulfonyl)phenyl]ethanone.

20. The method according to claim 19, **characterized in that** in the step (3), 1-{2-chloro-3-[(*RS*-tetrahydrofuran-2-ylmethoxy)methyl]-4-(methylsulfonyl)phenyl}ethanone is subjected to an oxidation reaction with an oxidizing agent to obtain 2-chloro-3-[(*RS*-tetrahydrofuran-2-ylmethoxy)methyl]-4-(methylsulfonyl)benzoic acid.

21. The method according to claim 20, **characterized in that** the oxidation reaction is carried out in the presence of a catalyst; the catalyst is one or more of cobalt acetate, manganese acetate, copper acetate, vanadium pentoxide, selenium dioxide, ruthenium on carbon, and ruthenium dioxide; and the oxidizing agent is one or more of oxygen, compressed air, ozone, hydrogen peroxide, nitric acid, manganese dioxide, sodium periodate, and potassium periodate.

22. The methtod according to claim 21, **characterized in that** the catalyst is used in an amount of 0.001-0.5 mol relative to 1 mol of 1-{2-chloro-3-[(*RS*-tetrahydrofuran-2-ylmethoxy)methyl]-4-(methylsulfonyl)phenyl}ethanone; and the oxidizing agent is used in an amount of 1-10 mol, preferably 1-5 mol, relative to 1 mol of 1-{2-chloro-3-[(*RS*-tetrahydrofuran-2-ylmethoxy)methyl]-4-(methylsulfonyl)phenyl}ethanone.

23. The method according to any one of claims 14-19, **characterized in that** in the step (3), 1-{2-chloro-3-[(*RS*-tetrahydrofuran-2-ylmethoxy)methyl]-4-(methylsulfonyl)phenyl} ethanone is subjected to a haloform reaction with a haloform reaction reagent, followed by acidification by addition of an aqueous solution of an acid to obtain 2-chloro-3-[(*RS*-tetrahydrofuran-2-ylmethoxy)methyl]-4-(methylsulfonyl)benzoic acid.

24. The method according to claim 23, **characterized in that** the haloform reaction reagent is selected from one or more of sodium hypochlorite, sodium hypobromite, liquid bromine and sodium hydroxide; and the haloform reaction reagent is used in an amount of 3-4.5 mol in terms of effective halogen relative to 1 mol of 1-{2-chloro-3-[(*RS*-tetrahydrofuran-2-ylmethoxy)methyl]-4-(methylsulfonyl)phenyl}ethanone.

25. The method according to claim 23 or 24, **characterized in that** in the step (3), the acid is one or more of hydrochloric acid, sulfuric acid, acetic acid, formic acid and phosphoric acid.

26. A compound, selected from the following compounds:

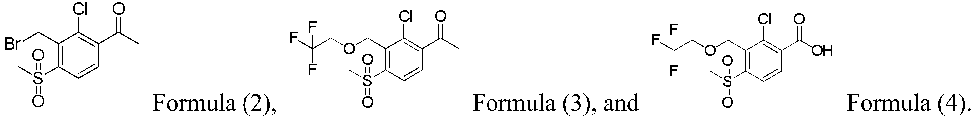

27. A compound, selected from the following compounds:

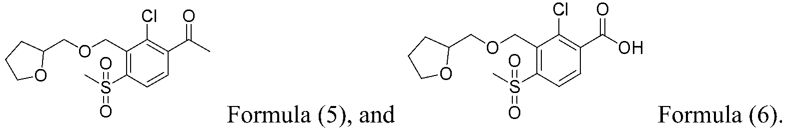

28. Use of a compound of a formula (2) and a compound of a formula (3) in the preparation of tembotrione, **characterized in that** structures of the formula (2) and the formula (3) are as follows:

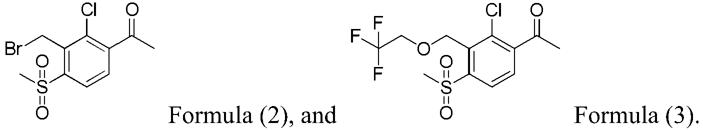

29. Use of a compound of a formula (2) and a compound of a formula (5) in the preparation of tefuryltrione, **characterized in that** structures of the formula (2) and the formula (5) are as follows:
